# EUROPÄISCHE PATENTSCHRIFT

(11) **EP 2 897 662 B1**
(45) Veröffentlichungstag und Bekanntmachung des Hinweises auf die Patenterteilung: **06.05.2020**
(21) Anmeldenummer: 13771060.4
(22) Anmeldetag: 20.09.2013
(51) Int. Cl.: A61M 1/00, A61M 27/00, A61F 13/00

(54) **UNTERDRUCKBEHANDLUNGSANORDNUNG UND FOLIE ZUR HERSTELLUNG EINER UNTERDRUCKBEHANDLUNGSANORDNUNG**
VACUUM TREATMENT ARRAY AND FILM FOR PRODUCING A VACUUM TREATMENT ARRAY
DISPOSITIF DE TRAITEMENT PAR PRESSION NÉGATIVE ET FEUILLE POUR LA RÉALISATION D'UN DISPOSITIF DE TRAITEMENT PAR PRESSION NÉGATIVE

(30) Priorität: 20.09.2012 DE 102012018598; 06.12.2012 DE 102012024001; 27.12.2012 DE 102012025388; 07.01.2013 DE 102013000047
(43) Veröffentlichungstag der Anmeldung: 29.07.2015
(60) Teilanmeldung: 20163582.8
(73) Patentinhaber: Lohmann & Rauscher GmbH, 2525 Schönau/Triesting (AT)
(72) Erfinder: Loske, Gunnar, 22926 Ahrensburg (AT)
(74) Vertreter: Seranski, Klaus
(86) Internationale Anmeldenummer: PCT/EP2013/002839
(87) Internationale Veröffentlichungsnummer: WO 2014/044400

(56) Entgegenhaltungen:
- EP-A1- 2 394 677
- WO-A1-2011/038949
- WO-A1-2013/025285
- DE-A1-102008 061 536
- DE-A1-102011 013 743
- US-A1- 2002 161 346
- US-A1- 2003 109 855
- US-A1- 2010 049 166
- US-A1- 2010 160 877

## Beschreibung

Die Erfindung betrifft eine Unterdruckbehandlungsanordnung (siehe z.B. US 2010/0049166) mit mindestens einem offenporigen Kontaktelement, über das ein Unterdruck und/oder Sog in einer Körperhöhle erzeugt werden kann.

Die konventionelle Unterdruckbehandlung bzw. Vakuumtherapie (Niederdruck-Wundtherapie) wird zur Behandlung von äußeren Wunden eingesetzt. Ein offenporiger Polyurethanschwamm oder ein offenporiges Fluidsammelmittel wird in die Wunde eingelegt, mit einer Folie versiegelt und dann unter einen Unterdruck gesetzt. Unter der Folie können unterstützt durch den Unterdruck die Wundsäuberung und Wundheilung stattfinden.

Die endoskopische Vakuumtherapie bzw. Unterdruckbehandlung wird zur Behandlung von inneren Wunden eingesetzt. Ihre Effektivität konnte zunächst bei Nahtundichtigkeiten am Enddarm, dann auch bei Darmleckagen anderer Lokalisation sowie im Bereich der Speiseröhre, des Magens, des Dünn- und Dickdarms nachgewiesen werden. Bei inneren, unter der Hautoberfläche liegenden Wunden, Hohlräumen, Abszessen, Empyemen, Fisteln od. dgl., die über eine Öffnung nach außen endoskopisch zugänglich sind oder zugänglich gemacht werden, kann die endoskopische Vakuumtherapie zur Wundbehandlung eingesetzt werden. Bei der endoskopischen Vakuumtherapie werden die natürlichen oder künstlichen Zugangswege zu Hohlorganen, Magen-Darmtrakt und Körperhöhlen endoskopisch genutzt.

Es werden offenporige Polyurethanschwammdrainagen unter Verwendung von Endoskopen innerlich, intracorporal, intraluminal und intracavitär eingebracht. Bei der intraluminalen Therapievariante wird der Schwammkörper in einem Darmlumen in Defekthöhe platziert. Bei der intrakavitären Variante wird der Schwammkörper durch den Defekt hindurch in eine extraluminale Wundhöhle eingebracht.

Im Rahmen der Beschreibung dieser Erfindung werden intraluminale Bereiche ebenso wie extraluminale Bereiche als Körperhöhle bezeichnet.

Die beiden vorstehend angegebenen Therapien können auch kombiniert werden. Nach Positionierung des Schwammkörpers wird über den ausgeleiteten Drainageschlauch ein Unterdruck bzw. Sog angelegt. Die Körperhöhle (Wundhöhle bzw. das Darmlumen) kollabiert unter dem Sog gemeinsam mit dem elastischen Schwammkörper. Die Schwammoberfläche saugt sich an der Wundoberfläche saugnapfartig an. Gleichzeitig fixiert sich der Schwamm so auch durch den Sog am Platzierungsort. Es findet eine effektive Wunddrainage statt. Gleichzeitig wird der Wunddefekt verschlossen. Unter der dauerhaften Drainagewirkung und Vakuumausübung an der Wundfläche reinigt sich die Wunde, es bildet sich Granulationsgewebe und die Wunde verheilt sekundär. Im mehrtätigen Intervall wird ein endoskopischer Wechsel der Schwammdrainage vorgenommen.

Im Rahmen der Erfindung wird eine entsprechende Schwammdrainage auch als Kontaktelement bezeichnet.

Zur Platzierung einer Schwammdrainage bzw. eines Kontaktelements im Enddarm zur Behandlung von postoperativen Anastomoseninsuffizienzen existiert ein zugelassenes Platzierungssystem.

Zur Platzierung der Kontaktelemente bzw. Schwammdrainagen in tiefer liegenden Körperregionen, wie bspw. dem Dickdarm, der Speiseröhre oder dem Zwölffingerdarm, mit teilweise kurvenreichen Zugangswegen werden Schwammdrainagen benutzt, die aus einem Drainageschlauch bestehen, an dessen Ende das Kontaktelement angenäht wird. Der entsprechende Schwammkörper wird mit Greifzangen, Polypengreifern oder Schlingen gegriffen und unter endoskopischer Führung eingebracht.

Zur Ableitung von Wundsekreten, Körperflüssigkeiten, Vereiterungen und nach Operationen werden Drainageschläuche eingelegt. Es handelt sich hierbei um Schläuche, in deren inneres Lumen durch seitliche Perforationsöffnungen Sekrete oder Gase abgeleitet werden können. Die Ableitung kann als Schwerkraftdrainage, Überlaufdrainage, Kapillardrainage oder unter Sog erfolgen. Drainagen können als Schlauchdrainage oder auch als flächige Drainage konstruiert sein. Spezielle Drainagen, bspw. zur Ableitung eines Gallenstaus, werden auch operativ oder endoskopisch eingelegt. Über Drainagen kann auch gespült werden. An Drainagen kann ein Unterdruck angelegt werden.

Wunddrainagen entfalten ihre Wirkung meist nur unmittelbar nach einer Operation, da es u. a. durch Fibrinausfällungen, Koagulation von Blut und dem Anliegen von Gewebe zu einer schnellen Verstopfung der Drainageöffnungen kommt. Ob eine Drainage möglich ist, hängt auch von der Beschaffenheit des abzuleitenden Materials ab. Stuhl, Speichel oder Eiter sind zähflüssig und benötigen relativ großlumige Perforationsöffnungen, während Urin, Ascites, Galle u. dgl. sehr flüssig sind und auch durch kleinlumige Öffnungen abgeleitet werden können.

Die herkömmlichen Drainagen bestehen aus einem Schlauch, an dem sich eine oder mehrere seitliche Perforationsöffnungen befinden. Die Öffnungen kommunizieren fluidleitend direkt mit dem inneren Lumen der Drainage.

Bei einer offenporigen Schwammdrainage bzw. einem offenporigen Kontaktelement wird ein mit Perforationsöffnungen ausgerüsteter Drainageschlauch in einen offenporigen Schwamm eingeführt. Der Drainageschlauch ist über die Perforationsöffnungen fluidleitend mit dem offenporigen Fluidsammelelement verbunden. Der Schwamm wirkt wie ein Filter. Durch die offenporige Schwammstruktur kann sich die Schwammoberfläche bei Anlegen eines Unterdrucks breitflächig an eine Wunde bzw. die Wundränder ansaugen. Die offenporige Struktur des Schwamms ermöglicht ein Ansaugen von zahlreichen Porenöffnungen über eine große Fläche. Die Porenöffnungen des Schwamms wirken wie kleine Saugnäpfe. Durch die offenporige Struktur des Fluidsammelmittels bzw. Kontaktelements kommunizieren die zahlreichen Perforationsöffnungen fluidleitend untereinander. Hierdurch wird die Aufrechterhaltung eines angelegten Unterdrucks an der anliegenden Wundoberfläche auch bei Verstopfen einzelner Poren sichergestellt. Wenn auch nur über eine kleine Oberfläche ein Sekret abgesaugt werden kann, wird durch die offenporige Kommunikation der unzähligen Poren untereinander diese Drainage auch sichergestellt. Dieses ist ein wesentlicher Unterschied zu den konventionellen Drainageschläuchen, bei denen ein Schlauch einzelne Perforationsöffnungen aufweist. Sobald diese Öffnungen verstopft sind, ist wegen der fehlenden Verbindung zwischen den einzelnen Perforationsöffnungen (außer durch das Innenlumen des Schlauchs) eine Sog- und Drainagewirkung unterbrochen.

In der EP-A-12001013.7 ist eine Unterdruckbehandlungsanordnung beschrieben, bei der die Kontaktelemente durch zwei offenporige Drainageschichten gebildet sind. Die Poren der offenporigen Drainageschichten kommunizieren über den dazwischen liegenden Drainageraum miteinander. Zwischen die beiden Drainageschichten ist eine mit einer Absaugeinrichtung verbindbare Sauganordnung zum Absaugen von zwischen die Drainageschichten gelangendem Exsudat angeordnet. Zur Förderung der Wundheilung ist der Sauganordnung dieser bekannten Unterdruckbehandlungsanordnung eine Spülanordnung zum Zuführen eines Fluids zwischen die Drainageschichten zugeordnet. Durch die Spülanordnung strömt ein Fluid in die Körperhöhle ein. Dadurch wird kontinuierlich der Fluß in der Sauganordnung aufrechterhalten. Koagulationen werden vermieden, weil Grundexsudat kontinuierlich durch die Absauganordnung in Richtung auf die Absaugeinrichtung abgesaugt wird.

Der Offenbarungsgehalt dieser Schrift wird hinsichtlich der Ausführung der Drainageschichten und der zwischen den Drainageschichten angeordneten Absaugeinrichtung bzw. Spülanordnung ausdrücklich durch Inbezugnahme in diese Beschreibung aufgenommen.

Bei Einsatz herkömmlicher Unterdruckbehandlungsanordnungen der vorstehend beschriebenen Art bereitet die Platzierung, insbes. auch die endoskopische Platzierung, ebenso wie die Entfernung des Kontaktelements an bzw. von dem Behandlungsort in der Körperhöhle in vielen Fällen Probleme.

Angesichts dieser Probleme im Stand der Technik liegt der Erfindung die Aufgabe zugrunde, eine Unterdruckbehandlungsanordnung bereitzustellen, welche problemlos an einem Behandlungsort in einer Körperhöhle angeordnet und auch wieder von diesem Behandlungsort entfernt werden kann, sowie eine Folie zur Herstellung eines entsprechenden Kontaktelements anzugeben.

Die Erfindung ist definiert durch Anspruch 1. Erfindungsgemäß wird diese Aufgabe durch eine Weiterbildung der bekannten Unterdruckbehandlungsanordnungen gelöst, die im wesentlichen dadurch gekennzeichnet ist, daß das offenporige Kontaktelement zumindest abschnittweise schlauchartig mit einer eine Schlauchachse zumindest teilweise umlaufenden äußeren und/oder inneren Begrenzungsfläche ausgeführt ist.

Erfindungsgemäß kann das Kontaktelement, das bspw. auch als Fluidsammelelement zum Sammeln von Flüssigkeiten oder Gasen ausgeführt sein kann, hinsichtlich seiner Form und Beschaffenheit an den zum Anlegen des Unterdrucks und zum Ausleiten von Körperflüssigkeiten und Gas benötigten Drainageschlauch angepaßt werden. Es ist so ausgeführt, daß es bezüglich der Schlauchachse radial nicht über den Drainageschlauch hinaussteht. In diesem Fall kann es ohne Probleme in die Körperhöhle eingeführt werden bzw. auch wieder daraus entfernt werden.

Bei anderen Ausführungsformen der Offenbarung kann sich der Drainageschlauch im Bereich des Kontaktelements bzw. Fluidsammelelements erweitern, wobei sich eine stufenlose Erweiterung im Sinne einer einfachen Einführung bzw. Entfernung des Kontaktelements in bzw. aus der Körperhöhle als besonders vorteilhaft erwiesen hat. Das als Fluidsammelelement ausgeführte Kontaktelement kann fluidleitend derart mit einem kanalförmigen Lumen eines Drainageschlauchs verbunden sein, daß eine schlauchförmige Drainage gebildet ist, bei der das offenporige Fluidsammelelement als Teil der Schlauchwand und der ein Fluidkommunikationselement bildende Drainageschlauch in sich konstruktiv vereinigt sind.

Die Offenbarung geht insgesamt auf die folgenden Erkenntnisse zurück: Die endoskopische Platzierung und auch die Entfernung eines an einem Drainageschlauch angenähten offenporigen Polyurethanschwamms kann bedingt durch die Größe des Polyurethanschwamms sowie sein Volumen und seinen Durchmesser problematisch sein.

Durch die Inkongruenz der Durchmesser des als Kontaktelement dienenden Fluidsammelmittels bzw. Polyurethanschwamms und des Fluidkommunikationselements bzw. Drainageschlauchs kann die Platzierung und Entfernung der Drainage erschwert sein.

Bei der Platzierung und insbes. auch bei einer unbeabsichtigten unkontrollierten Entfernung eines herkömmlichen Unterdruckbehandlungssystems kann es bei der Nutzung im oberen Gastrointestinaltrakt mit Ausleitung aus dem Mund oder der Nase zu einer Verlegung der Atemwege und damit zu einer lebensbedrohlichen Situation für den Patienten kommen. Für die Platzierung und die Entfernung einer Drainage ist es vorteilhaft, wenn das Fluidsammelmittel bzw. Kontaktelement und das Fluidkommunikationselement bzw. der Drainageschlauch von gleichem Durchmesser sind und stufenlos ineinander übergehen.

Die Effektivität der Drainagewirkung einer Unterdruckdrainage ist nicht vom Volumen des Kontaktelements bzw. Schwammkörpers abhängig. Vielmehr kann mit einem im Vergleich zu der zu behandelnden Wundhöhle bzw. Körperhöhle vielfach kleineren Schwammkörper eine ebenso gute Wundheilung erzielt werden wie mit einem an die Größe der Körperhöhle angepaßten Kontaktelement, weil ein kleiner Schwammkörper zum Drainieren einer großen Wunde ausreichend sein kann und die Wunde mit dem Schwammkörper unter dem Sog über dem Schwammkörper kollabiert. Dabei wurde auch erkannt, daß die Wand eines Drainageschlauchs als offenporiges Kontaktelement bzw. Fluidsammelelement gefertigt werden kann, wobei der offenporige Wandbereich des Drainageschlauchs bzw. der durch das Kontaktelement gebildete Wandbereich des Drainageschlauchs nur wenige Millimeter dick sein muß, um als Vakuumschwammdrainage benutzt werden zu können. Der Drainageschlauch oder Teile des Drainageschlauchs können mit einer Krümmung (Pigtail) versehen werden.

Anders als ein an einen Drainageschlauch angenähter Schwamm, der sich im Rahmen der Unterdruckbehandlung an einer Wunde festgesaugt hat, kann ein erfindungsgemäß ausgeführtes Kontaktelement bei Entfernen desselben aus der Körperhöhle kaum noch vom Drainageschlauch abreißen. Im Rahmen der Erfindung können verschiedenartige, offenporige Fluidsammelelemente miteinander kombiniert werden, wobei die Platzierung der Drainagen und die Therapie mit speziell ausgerüsteten Drainagen und Zubehör vereinfacht werden kann.

Durch die Erfindung ergeben sich zahlreiche neue Therapiemöglichkeiten und Anwendungen, die insbes. bei der Wundbehandlung und im operativen Komplikationsmanagement nutzbar sind. Insbesondere wird bei Einsatz erfindungsgemäßer Unterdruckbehandlungsanordnungen, bei denen Kontaktelement und Kommunikationselement in einem Drainageschlauch konstruktiv vereinigt sind, das lebensbedrohliche Risiko der Verlegung der Atemwege, welches insbes. bei einer unbeabsichtigten Entfernung oder Dislokation bei der Anwendung im oberen Gastrointestinaltrakt auftreten kann, verhindert.

Bei einer bevorzugten Ausführungsform der Erfindung kommt eine schlauchförmige Drainage zum Einsatz, die in sich konstruktiv das Fluidsammelelement (Kontaktelement) und das Fluidkommunikationselement der Drainage vereinigt. Mit besonderem Vorteil können erfindungsgemäße Unterdruckbehandlungsanordnungen im Rahmen der endoskopischen Vakuumtherapie genutzt werden. Entsprechende Anordnungen lassen sich auch zur intraabdominellen, thorakalen Drainage nach Operationen, in der Wundbehandlung, Entlastung von Abszessen und bei Wundheilungsstörungen nutzen. Insbesondere kann eine erfindungsgemäße Unterdruckbehandlungsanordnung bei der Prophylaxe von Darmanastomosen und bei der Behandlung von Anastomoseninsuffizienzen und Perforationen am Darm eingesetzt werden. Das Anwendungsspektrum ist sehr weit.

Das Fluidkommunikationselement, welches gemäß einer bevorzugten Ausführungsform der Erfindung konstruktiv mit dem Fluidsammelelement vereinigt ist, wird im folgenden als Fluidkommunikationssammelelement bezeichnet und mit "FE" abgekürzt. Bei einem erfindungsgemäßen FE kann es sich um flexible unterdruckstabile Drainageschläuche handeln, bei denen die Wand oder Teile der Schlauchwand als offenporige Kontaktelemente bzw. Fluidsammelelemente konstruiert sind. So können durch die offenporigen Kontaktelemente als Bestandteil der Drainageschläuche Flüssigkeiten oder Gase abgeleitet werden.

Das FE ist fluidleitend mit offenporigen Fluidsammelsegmenten bzw. Kontaktelementen, die aus der Wand oder Teilen der Wand des FE bestehen, ausgerüstet. Die offenporigen Fluidsammelsegmente des FE befinden sich besonders vorteilhaft am distalen Ende des Schlauchs. Das offenporige Fluidsammelsegment bzw. Kontaktelement kann sich auch in einem Abschnitt zwischen dem proximalen oder distalen Ende des Schlauchs befinden. Bei dieser Ausführungsform der Erfindung befindet sich das Fluidsammelsegment zweckmäßigerweise im mittleren Abschnitt des FE.

Das FE kann mit nur einem oder auch mit zwei, drei oder mehr Fluidsammelsegmenten bzw. Kontaktelementen ausgerüstet sein. Fluidsammelsegmente bzw. Kontaktelemente können eine Länge von wenigen mm bis einigen cm aufweisen. Bei speziellen Indikationen, bspw. wenn eine vollständige Entlastung des Magens oder anderer langstreckiger Darmabschnitte erforderlich ist oder zur Sicherung und Deckung eines Defekts in der Speiseröhre, werden auch Fluidsammelelementabschnitte bzw. offenporige Kontaktelemente mit einer Länge von mehr als 20 cm, insbes. 30 cm oder mehr, eingesetzt. Die Drainage eignet sich insbes. auch zur zusätzlichen Sicherung einer kritischen Anastomosensituation bei Nähten am gesamten Magen-Darm-Trakt zur Verhinderung einer postoperativen Anastomoseninsuffizienz.

Das Fluidsammelelement bzw. Kontaktelement kann aus einem offenporigen, elastischen, komprimierbaren Polyurethanschwammkörper bestehen. Vorzugsweise weist das Kontaktelement eine Porengröße von 200 µm bis 1000 µm auf, insbes. 400 µm bis 600 µm. Das Fluidsammelelement bzw. Kontaktelement kann auch aus einer ein-, zwei- oder mehrlagigen offenporigen Folie gebildet sein bzw. eine solche Folie aufweisen. Entsprechende Folien sind in der EP 2 427 477 A beschrieben. Der Offenbarungsgehalt dieser Schrift wird hinsichtlich der Ausführung offenporiger, mehrlagiger Folien hiermit durch ausdrückliche Inbezugnahme in diese Beschreibung aufgenommen.

Im Rahmen der Erfindung ist auch an den Einsatz einer Kombination aus einem offenporigen Polyurethanschwammkörper und einer offenporigen Folie zum Erhalt eines offenporigen Fluidsammelelements bzw. Kontaktelements gedacht. Das Fluidsammelelement kann aus einem offenporigen Kunststoffmaterial bestehen. Bevorzugt werden als Material für das FE Polyurethan, Polyvinyl und Polyethylen verwendet. Wenn das Fluidsammelsegment bzw. Kontaktelement äußerlich mit einer offenporigen Folie beschichtet ist, soll hierdurch die Gleiteigenschaft des FE verbessert werden. Die Konstruktion mit offenporigen Folien als Kontaktelement ermöglicht auch einen Aufbau mit minimalem Durchmesser bei guter Fluidleitung. Des weiteren erhöht die Ausrüstung mit Folien die Reißfestigkeit des FE. Das als Fluidsammelelement ausgeführte Kontaktelement ist zweckmäßigerweise fluidleitend mit dem Fluidkommunikationselement bzw. Drainageschlauch verbunden. Das Fluidkommunikationselement besteht vorzugsweise aus einem Drainageschlauch mit einem zentralen fluidleitenden Kanal, welcher als Schlauch in das Fluidsammelelement bzw. Kontaktelement weitergeführt ist und hier über seitliche Öffnungen fluidleitend mit dem offenporigen Fluidsammelelement, welches Teil der Wand des Fluidkommunikationselements ist, verbunden ist. Das Fluidkommunikationselement bzw. der Drainageschlauch besteht vorzugsweise aus einem Schlauch mit einem zentralen Kanal und weiteren Kanälen, die sich in der Wandung des FE befinden und welche ebenfalls fluidleitend mit dem Fluidsammelsegment bzw. Kontaktelement verbunden sind. Das Fluidkommunikationselement kann aus einem Schlauch mit mehreren Kanälen bzw. Lumen bestehen.

Das Fluidkommunikationselement kann einen Drainageschlauch aufweisen, welcher aus einer Vielzahl fluidleitender Kanäle aufgebaut ist. Die fluidleitenden Kanäle können gleichlumig sein oder verschiedene Lumina besitzen. Die Kanäle können untereinander fluidleitend verbunden sein. Die Kanäle des Fluidkommunikationselements können zum Saugen und Spülen benutzt werden. In die Kanäle können ein Führungsdraht, Meßsonden oder Instrumente eingeführt werden. Die Kanäle können von der Länge und Anordnung im FE so bemessen sein, daß einzelne Kanäle mit einzelnen Fluidsammelelementen bzw. Kontaktelementen verbunden sind. Einer oder mehrere Kanäle in FE können auch so gestaltet sein, daß sie über das eigentliche FE mehrere cm bzw. dm hinausragen und bzw. als Ernährungssonde genutzt werden können. Das ist im besonderen bei der intraluminalen Anwendung der Sonde im oberen Gastrointestinaltrakt von Vorteil.

Bei einer bevorzugten Ausführungsform der Erfindung kann ein Führungsdraht in das FE eingebracht werden, über den das FE gleiten kann. Das FE kann insgesamt eine Länge von 80 cm bis 250 cm aufweisen.

Da auf das FE bei einem Durchzugsmanöver oder einem Entfernungsmanöver ein nicht unerheblicher Zug ausgeübt wird, ist es vorzugsweise zugfest und bruchfest konstruiert, damit es nicht abreißen kann. Vorzugsweise muß eine Zugfestigkeit von 50 N, insbes. 100 N, zweckmäßigerweise von bis zu 200 N gegeben sein. Das FE soll auch röntgendicht sein. Im Rahmen der Erfindung hat es sich als zweckmäßig erwiesen, wenn das FE in sich nicht abknicken kann, da bei einer Abknickung die Weiterleitung eines Unterdrucks bzw. der Abtransport von Sekreten unterbrochen ist.

Das aus der Körperhöhle ausgeleitete FE kann über Verbindungselemente mit einem unterdruckerzeugenden System, insbes. einer elektronischen Vakuumpumpe verbunden sein. Wenn sowohl der proximale als auch der distale Schenkel des Fluidkommunikationselements ausgeleitet ist, kann sowohl am distalen und/oder am proximalen Ende ein Unterdruck bzw. Sog angelegt werden. Es werden Unterdrücke im Bereich zwischen 40 mmHg und 200 mmHg angewendet. Bei der thorakalen Anwendung werden auch niedrigere Unterdrücke eingesetzt.

Vorzugsweise weist ein erfindungsgemäßes FE einen Außendurchmesser von 2 mm bis 20 mm auf. Bei einer besonders bevorzugten Ausführungsform der Erfindung kann eine mit einem erfindungsgemäßen offenporigen Kontaktelement ausgestattete Drainage mit einem geringen Durchmesser endoskopisch über den Arbeitskanal eines Endoskops platziert werden. Zweckmäßigerweise hat das FE in allen Abschnitten denselben Außendurchmesser. Das oder die offenporigen fluidleitenden Fluidsammelsegmente bzw. Kontaktelemente der Drainage gehen stufenlos in die Fluidkommunikationsabschnitte über. Dadurch wird es möglich, eine erfindungsgemäße Unterdruckbehandlungsanordnung bei einer Nutzung im oberen Gastrointestinaltrakt transnasal einzuführen. Mit bekannten Anordnungen ist das nicht möglich. Außerdem läßt sich die erfindungsgemäße Unterdruckbehandlungsanordnung bei Anpassen des Durchmessers des Kontaktelements an den Durchmesser des Drainageschlauchs ohne ein in der Kontaktelementkonstruktion bedingtes mechanisches Hindernis mit Zug leichter entfernen. Dadurch wird die Nutzung einer erfindungsgemäßen Unterdruckbehandlungsanordnung als cutan ausgeleitete Zieldrainage bei Operationen oder zur Ableitung von Flüssigkeiten in sämtlichen Körperhöhlen ermöglicht. Die Unterdruckbehandlung bzw. Vakuumtherapie kann an diesen Orten angewendet werden und die Entfernung der Drainage ist ohne erneuten operativen Eingriff möglich.

Über das FE kann im Rahmen der Erfindung auch eine Spülbehandlung vorgenommen werden. Insbesondere kann bei einer Platzierung des FE im mittleren Abschnitt und Ausleitung beider Fluidkommunikationsschenkel bzw. Drainageschläuche der eine Schenkel zur Saugung und der andere zur Spülung genutzt werden.

In die Wandung des FE können in Längsrichtung Drähte oder Fäden eingearbeitet sein, mit denen die Stabilität bzw. Zugfestigkeit des FE erhöht und somit ein Abreißen des FE verhindert werden kann.

An seinem distalen Ende ist das FE zweckmäßigerweise konisch ausgeführt und endet in einer Spitze. Dadurch wird das Platzierungsmanöver der Drainage erleichtert. Die konische Spitze der Drainage ist besonders vorteilhaft weich und atraumatisch gestaltet, um eine Verletzung von anliegendem Gewebe zu vermeiden.

An dem distalen Ende des Fluidkommunikationselements bzw. Drainageschlauchs, an dem Fluidsammelelement bzw. Kontaktelement oder in dem Fluidsammelelement ist vorteilhaft eine Vorrichtung befestigt, die mit einer Zange, Haken, Schlinge oder anderem Legeinstrument gefaßt werden kann. Insbesondere kann eine Faden- oder Drahtschlaufe befestigt sein. Besonders bevorzugt kann eine Greifperle aus Metall oder Kunststoff vorgesehen sein. Insbesondere kann eine Metall- oder Kunststofföse befestigt sein. Es kann auch ein Faden befestigt sein. Diese Vorrichtungen sind vorzugsweise zugfest gestaltet, so daß die Drainage bzw. Unterdruckbehandlungsanordnung an diesen Elementen durch Gewebe, Darmlumina und Fisteln gezogen werden kann. Die Vorrichtungen sind flexibel und atraumatisch gestaltet.

Die Platzierung einer erfindungsgemäßen Unterdruckbehandlungsanordnung kann mit einem Legeinstrument orthograd unter endoskopischer Sicht erfolgen. Bei Vorliegen einer zusätzlichen Verbindung nach außen kann mit dem Legeinstrument oder dem gefestigten Faden auch in der (Durch-)Zugtechnik platziert werden. Die Wechselmanöver können durch Nutzung der Durchzugtechnik erheblich vereinfacht werden.

Bei den bislang beschriebenen Ausführungsformen der Erfindung wird das offenporige Kontaktelement der erfindungsgemäßen Unterdruckbehandlungsanordnung in Verbindung mit einem Drainageschlauch zum Ableiten von Fluiden oder Gasen aus einer Körperhöhle eingesetzt. Zusätzlich oder alternativ kann die Unterdruckbehandlungsanordnung einen röhrenförmigen Hohlkörper für medizinische Anwendungen im menschlichen oder tierischen Körper aufweisen, dessen Außenseite mit dem Kontaktelement ausgestattet ist, bei der das Kontaktelement aus einer für Gase und Flüssigkeiten nicht durchlässigen Folie oder Membran besteht, deren nach außen liegende Seite eine offenporige Oberfläche aufweist, entlang der Flüssigkeiten und/oder Gase fließen und deren nach innen liegende Seite vorzugsweise eine nicht offenporige flüssigkeits- und/oder gasdichte Oberfläche aufweist.

Defekte von Darmwänden und Undichtigkeiten der Luftwege können zu schwersten Krankheitsbildern führen. Trotz aufwendiger operativer Verfahren und intensivmedizinischer Behandlung sind sie mit einer hohen Mortalität belastet.

Zur Überbrückung und Abdichtung von Defekten im Gastrointestinaltrakt werden als Alternative zur operativen Therapie selbstexpandierende Metall- und/oder Kunststoffstents eingesetzt. Die Stents können dabei mit einer gas- und/oder flüssigkeitsundurchlässigen Folienbeschichtung ganz oder teilweise beschichtet sein. Man spricht dann von gecoverten Stents. Durch die Beschichtung wird eine flüssigkeits- und gasdichte Abgrenzung zwischen dem Innenlumen und der Außenseite des Stents erreicht. Im Prinzip handelt es sich bei den Konstruktionen um selbstentfaltbare Hohlkörper oder Röhren, die über ein Legeinstrumentarium platziert werden.

Ebenso werden zur Abdichtung von Defekten Tuben eingesetzt, die im Prinzip aus einem zu beiden Enden offenen Kunststoffrohr bestehen. Stents und Tuben werden auch zur Überbrückung von lumenverlegenden Hindernissen, wie z. B. Krebsgeschwülsten, eingesetzt. Die Abdichtung durch einen gecoverten Stent wird bewirkt, wenn sich der Stent entfaltet und mit seiner Außenseite gegen die Darmwand drückt. Ein Nachteil von Stents liegt in der mangelhaften Abdichtung bei einer Lumeninkongruenz. Eine solche liegt immer vor, wenn bei einer Darmoperation verschiedene Lumen durch eine Naht vereinigt werden. Dieses ist z. B. bei einer Nahtverbindung von Speiseröhre und Magen der Fall. Wenn in diesem Bereich der Naht eine Undichtigkeit, bspw. im Übergang von Speiseröhre (kleinlumig) und Magen (großlumig), vorliegt, gelingt eine Abdichtung durch Entfaltung eines Stents meist nicht vollständig. Diese Situation entsteht häufig bei Anastomosensituationen. Dadurch kann die Behandlung von postoperativen Undichtigkeiten mit Stents und Tuben erschwert werden. Der Stent entfaltet sich nach der Auslösung und soll sich gegen die Darmwand drücken und sich dabei an dieser verankern und zur Schleimhaut abdichten, während ein Tubus nur eine Überbrückung im Verlauf des Lumens ohne einen Expansionsdruck nach außen auszuüben vornehmen kann.

Ein weiteres Problem von Stents und Tuben ist deren Dislokation. Diese tritt auf, wenn sich die Hohlkörper nicht ausreichend in der Darmwand verankern können.

Eine weitere Komplikation von Stents und Tuben ist die Perforation durch den im Darmlumen liegenden Hohlkörper durch die Wand von innen nach außen. Perforationen treten insbesondere an den trichterförmigen Erweiterungen der röhrenartigen Hohlkörper auf.

Eine neue Möglichkeit zur Behandlung von Undichtigkeiten, bspw. an der Speiseröhre, dem Magen oder durch zu hohe Dehnung, aber auch am Enddarm, besteht in der Methode der endoskopischen Unterdruckbehandlung bzw. Vakuumtherapie. Dabei werden offenporige Polyurethanschwammdrainagen intrakavitär und intraluminal endoskopisch eingebracht und über eine Drainageleitung unter einen Unterdruck gesetzt. Durch die Sogwirkung kommt es zur Ansaugung des Schwammkörpers an der Darmwand mit Abdichtung des überdeckten Defekts und Induktion einer sekundären Wunde, die dann selbst abheilen kann.

Gemäß diesem Gesichtspunkt der Erfindung wird vorgeschlagen, die technischen Vorzüge der Vakuumtherapie mit einem Stent bzw. Tubus durch die Nutzung einer einseitig offenporigen Folie bzw. eines einseitig offenporigen Kontaktelements zu verbinden. Dabei werden die vorstehend beschriebenen Nachteile im Rahmen der Erfindung beseitigt bzw. entsprechende Probleme gelöst. Die Patientensicherheit wird durch Vermeidung von stentbedingten Komplikationen erhöht und das Indikationsspektrum der Therapie erweitert. Zahlreiche neue Therapiemöglichkeiten werden eröffnet und insbes. im Management von operativen und endoskopischen Komplikationen können Stent und Tubus zum Einsatz kommen. Die Anwendung soll im menschlichen und tierischen Körper möglich sein.

Die Ummantelung eines selbstexpandierenden Metall- und Kunststoffgitterstents wird gemäß diesem Gesichtspunkt der Erfindung mit einem offenporigen Kontaktelement in Form einer einseitig offenporigen Folie vorgenommen, indem die Form der als Kontaktelement eingesetzten Folie entsprechend der Form des Stents oder Tubus eine Schlauchachse zumindest teilweise umlaufend ausgeführt ist. Die das Kontaktelement bildende Spezialfolie kann aus einer für Gas und Flüssigkeit nicht durchlässigen Membran bestehen. Diese Membran hat zwei Seiten, die sich hinsichtlich ihrer Eigenschaften voneinander unterscheiden.

Die eine Seite der Membran ist nicht offenporig. Diese Seite kommt auf den Metallgitterdrähten bzw. dem Kunststoffgitter des Stents zu liegen und wird mit diesem konstruktiv verbunden. Das durch die Folie gebildete Kontaktelement kann durch Kleben und/oder Verschweißen fest mit den Drähten oder dem Gitter verbunden werden. Die Folie bildet die Innenseite des röhrenförmigen, an beiden Enden offenen Hohlkörpers.

Die andere Seite der Membran bildet die Außenseite dieses röhrenförmigen Hohlkörpers. Sie hat eine offenporige Oberfläche. Diese Oberfläche ist dadurch gekennzeichnet, daß entlang dieser Folienseite bzw. dieser Seite des Kontaktelements Gase und Flüssigkeiten frei kommunizieren, sich bewegen und fließen können. Durch die offenporige Oberflächenstruktur hat die Folie bzw. das Kontaktelement auf dieser Seite die Eigenschaften eines Fluidsammelelements. An diese offenporige Oberflächenseite kann ein Unterdruck angelegt werden. Wenn an diese Seite der Unterdruck angelegt wird, wird durch die offenporige Konstruktion ein zur Unterdruckquelle gerichteter Sog über die gesamte offenporige Folienoberfläche ermöglicht. Diese offenporige Folienseite bzw. diese offenporige Seite des Kontaktelements gelangt in Kontakt mit dem umliegenden Gewebe und saugt sich durch einen Unterdruck an das Gewebe an.

Auf diese Weise kann die mangelhafte Abdichtung von konventionellen gecoverten Stents, z. B. bei einer Inkongruenz von Darmlumina, ausgeglichen werden. Die Außenseite des Stents haftet mit Hilfe des (einseitig) offenporigen Kontaktelements unter Sog wie ein Saugnapf an der Darmwand an. Der Stent wird so am Platzierungsort fixiert und eine Dislokation, die eine typische Komplikation beim Einsatz herkömmlicher Stents ist, wird verhindert. Erfahrungsgemäß kommt es nicht darauf an, größere Flüssigkeitsmengen über den Unterdruck zu drainieren, sondern darauf, eine innige Verbindung durch den Sog zwischen der Darmwand und dem Stent herzustellen.

Vorzugsweise geht im proximalen und/oder distalen Randbereich des Kontaktelements bzw. der Folienummantelung die offenporige Außenseite in eine nicht offenporige Oberflächenstruktur über, so daß hierdurch im Randbereich der Folie eine nicht fluidleitende Begrenzung gebildet wird. Dadurch wird der Aufbau eines an der offenporigen Oberfläche angelegten Unterdrucks erleichtert. Die Offenporigkeit der Folienseite bzw. Kontaktelementseite kann durch eine verschiedenartige Gestaltung der Oberflächenstruktur der offenporigen Folienseite erreicht werden. Die Offenporigkeit kann bspw. durch gitter-, blasen-, noppen-, finger- oder kanalförmige Strukturen erreicht werden. Die Porengröße soll zwischen 200 µm und 1000 µm liegen. Der Unterdruck wird vorzugsweise mit Hilfe einer elektrisch steuerbaren Pumpe erzeugt. Der Unterdruck kann allerdings auch mit Hilfe einer Vakuumflasche erzeugt werden. Der notwendige Unterdruck liegt nach den Kenntnissen aus der endoskopischen Vakuumschaumtherapie vorzugsweise zwischen 40 mmHg und 200 mmHg.

Der Unterdruck kann mit einem Fluidkommunikationselement weitergeleitet werden, welches vorzugsweise aus einem oder mehreren unterdruckstabilen Schläuchen besteht, die fluidleitend mit der offenporigen Seite des Kontaktelements verbunden sind. Das Fluidkommunikationselement kann sich fächerförmig oder wurzelartig auf der offenporigen Folienseite verzweigen. So kann die Saugwirkung auf der gesamten Oberflächenseite optimiert werden. Das Fluidkommunikationselement kann entfernbar, d. h. von dem röhrenartigen Hohlkörper (Tubus oder Stent) lösbar, konstruiert sein, so daß der Stent auch ohne Unterdruckbeaufschlagung genutzt werden kann. Durch die Entfernbarkeit ist es möglich, in den ersten Tagen der Therapie die Vakuum- bzw. Unterdruckbehandlung durchzuführen und dann den Sog zu beenden, den Stent aber noch am Behandlungsort zu belassen. Durch diese Eigenschaft ist es möglich, die Konfiguration von Stents zu variieren. Eine typische Konfiguration von Stents ist die tulpenförmige, trichterartige Öffnung des Lumens nach außen. Dadurch soll eine bessere Abdichtung und Verankerung des Stents an der Wand erreicht werden.

Als Komplikation beim Einsatz von Stents werden häufig Perforationen durch diese tulpenförmigen Erweiterungen beobachtet. Bei einem erfindungsgemäß ausgestatteten Stent mit einer einseitig offenporigen Oberfläche ist die Anhaftung an der Wand durch den Unterdruck gesichert, so daß die Trichterform minimiert werden kann. Bei einer bevorzugten Ausführungsform der Erfindung wird vollständig auf die Trichterform verzichtet. Dadurch wird die Patientensicherheit bei der Anwendung von Stents im Gastrointestinaltrakt erheblich erhöht. Stentbedingte Komplikationen durch Perforationen und Dislokationen können verhindert werden. Gleichzeitig wird die Wirksamkeit des Stents optimiert. Das Kontaktelement ist bei diesem Gesichtspunkt der Erfindung zweckmäßigerweise als Folie ausgeführt. Die Folie kann dünnwandig und/oder elastisch und/oder flexibel und/oder durchsichtig sein.

Im Rahmen des erfindungsgemäßen Einsatzes röhrenförmiger Hohlkörper mit offenporigen Kontaktelementen wird insbes. auch die Ausübung der Vakuum- bzw. Unterdrucktherapie am Bronchialsystem bei Trachea- oder Bronchusverletzungen ermöglicht. Dadurch werden ganz neue Therapieoptionen für diese schwer zu behandelnden Krankheitsbilder geschaffen. Die Ausübung der endoskopischen Vakuumtherapie ist für diese Indikation bislang nicht möglich. Es ist vorstellbar, daß zahlreiche Operationen durch den Einsatz dieser neuartigen Behandlungsmöglichkeit vermieden werden können.

Die vorangegangenen Darstellungen gelten in gleicher Weise für einen Tubus, welcher aus einem beidseits offenen Kunststoffrohr besteht, dessen äußere Oberfläche mit einer einseitig offenporigen Folie bzw. einem einseitig offenporigen Kontaktelement schlauchförmig ummantelt wird.

Eine Sonderform eines einseitig offenporigen Tubus stellt ein Tubus dar, in dem die Tubuswandung des Tubus selbst mit den Eigenschaften der einseitig offenporigen Folie bzw. des einseitig offenporigen Kontaktelements konstruiert wird. Das bedeutet, daß der Tubus aus einem hohlen Rohr besteht, bei dem die Wand einseitig offen ist. Die Wand ist nicht permeabel für Gas und Flüssigkeit. Das innen liegende Lumen ist nicht offenporig. Die äußere Oberflächenseite der Röhre ist in ihrer Oberflächenstruktur offenporig konstruiert und als offenporiges Kontaktelement ausgeführt.

Ein besonderes Ausführungsbeispiel eines einseitig offenporigen Tubus stellt ein Overtube-Tubus für Endoskope dar. Der Overtube kann vorteilhaft mit einem kompletten Längsschlitz versehen sein.

Ein besonderes Ausführungsbeispiel eines einseitig offenporigen Tubus stellt ein ein- oder doppellumiger endotrachealer Intubationstubus dar. Alternativ oder zusätzlich zur trachealen Abdichtung kann sich der Tubus endotracheal über ein oder mehrere offenporige(s) zirkuläre Tubussegment(e) unter Unterdrucksog an die Trachealwand ansaugen.

Eine zur Herstellung eines offenporigen Kontaktelements einer Unterdruckbehandlungsanordnung verwendete erfindungsgemäße Folie ist im wesentlichen dadurch gekennzeichnet, daß sie aus einer für Gase und Flüssigkeiten nicht permeablen Membran besteht, deren eine Seite eine nicht offenporige Oberfläche aufweist und deren andere Seite eine offenporige Oberfläche aufweist, entlang welcher Flüssigkeiten und/oder Gase fließen können. Die nicht offenporige Oberfläche einer Folie kann zumindest abschnittweise glatt gestaltet sein. Zusätzlich oder alternativ kann sie auch profilierte Oberflächenbereiche aufweisen. Insbesondere kann diese Oberfläche mit einem rinnenartigen Profil oder mit einem Gitterprofil ausgestattet sein.

Die andere Seite der Folie hat eine offenporige Struktur. Die Offenporigkeit dieser Oberfläche ist dadurch gekennzeichnet, daß sich Flüssigkeiten oder Gase entlang dieser Oberfläche frei in alle Richtungen bewegen können und miteinander kommunizieren. Wenn die offenporige Oberflächenseite auf ein Körpergewebe aufgelegt wird, können sich Flüssigkeiten und Gase durch die Offenporigkeit zwischen der Gewebeoberfläche und der Folienoberflächenseite bewegen. Es kann ein Unterdruck in dem Zwischenraum zwischen dem Gewebe und/oder an der offenporigen Oberflächenseite angelegt werden. Es kann ein gerichteter Unterdruck in dem Raum angelegt werden. Damit ist gemeint, daß die Flüssigkeiten oder Gase von einer oder mehren Unterdruckquellen angesaugt werden können und sich in Richtung auf diese Quellen bewegen. Entlang dieses Raums der offenporigen Oberflächenseite der Folie können Flüssigkeiten und Gase durch einen Unterdruck gerichtet strömen. Flüssigkeiten und Gase können ebenso umgekehrt in diesen Raum von außen eingeleitet werden. Beispielsweise kann ein flüssiges Medikament zugeführt werden. Die offenporige Oberflächenseite kann als Medikamententräger fungieren und mit speziellen Stoffen, bspw. Antiseptika, beaufschlagt werden. Durch einen angelegten Unterdruck kann sich diese Folienseite breitflächig über die gesamte Oberfläche an Körpergewebe oder andere geschlossene Oberflächen ansaugen. Die Offenporigkeit bleibt bei Ausübung des Unterdrucks erhalten.

Die offenporige Struktur der Oberfläche ist konstruktiver Teil der Folie selbst. Die Folie vereinigt in sich die Offenporigkeit und die Nichtoffenporigkeit bzw. Undurchlässigkeit auf der anderen Seite.

Die Offenporigkeit wird durch eine offenporige Oberflächenstruktur der Folie selbst erlangt. Die offenporige Oberflächenstruktur kann insbes. durch eine offenporige gitterförmige Oberflächenstruktur erreicht werden. Sie kann durch eine noppenartige und/oder zottenförmige Struktur oder eine Kombination aus unterschiedlichen Oberflächenmustern erreicht werden.

Die Offenporigkeit der Oberfläche kann erreicht werden, indem die Membran bspw. mit einem offenporigen Stoff beaufschlagt wird. Die Oberfläche kann insbes. mit einer dünnen Lage eines offenporigen Fluidsammelelements beaufschlagt sein. Das Fluidsammelelement kann insbes. mit einer Lage von offenporigem Polyurethanschaum beaufschlagt sein.

Die Offenporigkeit der einen Folienseite kann auch dadurch erreicht werden, daß die Folie auf der offenporigen Seite aus offenporigen doppel- oder mehrlagigen perforierten Folien, bspw. gemäß EP-A-2424477, besteht. Diese mehrlagigen Folien sind untereinander durch Abstandhalter so beabstandet, daß die Membranen keinen direkten flächenhaften Kontakt zueinander haben. Die Folienmembranen der mehrlagigen offenporigen Folien sind mit einer Vielzahl kleiner Perforationsöffnungen versehen. Diese Perforationsöffnungen können in einem geordneten Muster angelegt sein oder sie können auch unregelmäßig verteilt sein.

Bei einer mehrlagigen Folienlage können die Folien im Randbereich der Folie vorteilhaft miteinander nicht fluidleitend verschweißt sein, so daß die Fluidleitung nicht über den Rand hinaus möglich ist. Bei einer einlagigen einseitig offenporigen Folie gehen vorteilhaft beide Folienseiten beidseits in eine nicht offenporige Oberflächenstruktur der Folienseite über. Hierdurch ist die einseitig offenporige Folie im Randbereich nicht fluidleitend.

Der Randbereich kann je nach Anwendung sowohl von der glatten nicht offenporigen Seite mit einem Klebstoff ausgerüstet sein, als auch von der offenporigen Seite. Die Folie kann auf diese Weise wie ein Pflaster auf eine Wunde aufgeklebt werden, diese abdichten und verschließen.

Durch die fluidleitende Verbindung mit einem Fluidkommunikationselement, welches fluidleitend mit der offenporigen Folienseite verbunden ist, kann mit einem unterdruckerzeugenden System, bspw. einer elektronischen Pumpe oder einer Vakuumflasche, ein Unterdruck auf der offenporigen Folienseite erzeugt werden. Das Fluidkommunikationselement kann aus schlauchförmigen Drainageleitungen bestehen. Die schlauchförmigen Fluidkommunikationselemente können in die Folie integriert sein und fluidleitend mit der offenporigen Folienseite verbunden sein. Die Fluidkommunikationselemente können sich kapillarartig auf der offenporigen Oberfläche verzweigen. Es kann auch die geschlossene Folienseite fluidleitend eröffnet werden und über diese Öffnung das Fluidkommunikationselement fluidleitend mit der offenporigen Folienseite verbunden werden. Dieses kann durch eine fluidleitende Pelotte, die auf die Folie aufgeklebt wird, vorgenommen werden.

Bei einer bevorzugten Ausführungsform kann der Randbereich auch fluidleitend offenporig sein. Das kann bspw. bei der Anwendung im offenen Abdomen vorteilhaft sein, wenn die Folie benutzt wird, um ein Organ (z. B. Milz oder Leber) zu umhüllen und mit der offenporigen Seite unter einen Unterdruck zu setzen. Die Folie wird sich dann auch im Randbereich durch Ansaugen von Gewebe verschließen. Dadurch, daß bei dieser Anwendung kein geschlossener Randbereich notwendig ist, kann die Folie frei zugeschnitten und an die Erfordernisse angepaßt werden. Dadurch, daß die Folie einseitig offenporig ist, wird die Saugwirkung nur an dieser Seite entfaltet, während die an der geschlossenen Seite anliegenden Organe nicht dem Sog ausgesetzt sind. Hierdurch wird eine Verletzung durch den Unterdruck der nicht einer Behandlung bedürfenden Organe verhindert. Das ist ein besonderer Vorteil.

Die Folie ist insbes. faltbar und/oder weich und atraumatisch und/oder elastisch und/oder durchsichtig und/oder nicht durchsichtig und/oder farbig. Die Folie kann genäht, geschweißt und geklebt werden.

Die Folienstärke beträgt zweckmäßigerweise 0,5 mm bis 5 mm. Wenn die Folie benutzt wird, um ein medizinisches Hilfsmittel auszurüsten, bspw. einen selbstexpandierenden Stent, kann die Folienstärke auch noch dünner gewählt sein, damit der Stent möglichst klein komprimiert werden kann.

Die Folie kann fluidleitend gemeinsam mit anderen Fluidsammelelementen verbunden werden. Sie kann bspw. bei einem Okklusionsverband oder bei der Niederdruckwundtherapie an äußeren Wunden als Folie benutzt werden.

Die Folie kann zu einer mehrlagigen Folie gefaltet oder gerollt werden. Sie kann in Verbindung mit einem Fluidkommunikationselement als aktive Unterdruckfolie und/oder aktive Folie eingesetzt werden.

Die Folie kann an verschiedene Körperformen angepaßt werden. Beispielsweise kann aus der Folie ein Handschuh oder eine Gesichtsmaske hergestellt werden, so daß ein der Körperform angepaßter Wundverband wie ein Kleidungsstück angelegt werden kann.

Im Randbereich ist die Folie vorteilhafterweise mit einer Klebemöglichkeit versehen, so daß der Verband an der Haut verklebt werden kann.

Über ein Fluidkommunikationselement, welches mit der offenporigen Folienseite fluidleitend verbunden ist, kann ein Unterdruck an der Wunde bzw. Haut angelegt werden. Durch den Unterdruck entsteht sowohl eine Sogwirkung als auch eine Druckwirkung auf das anliegende Gewebe. Durch den Unterdruck wird das in typischer Weise bei einer Wunde vorliegende Wundsekret drainiert, so daß die Wunde unter leichter Kompression drainiert ist.

Besonders bevorzugt ist die Folie durchsichtig gestaltet, so daß die Begutachtung einer oberflächlichen Wunde durch den Verband hindurch erfolgen kann. Wenn die Folie locker auf einer Hautoberfläche oder Wunde aufgelegt wird und elastisch ist, wird sie durch den anliegenden Unterdruck bis auf die Gewebeoberfläche gezogen und paßt sich dieser an.

Ein weiteres typisches Anwendungsbeispiel ist die Wundversorgung nach Hauttransplantationen.

Mit einer Folie können insbes. auch medizinische Geräte oder therapeutische Hilfsmittel technisch ausgerüstet werden. Vorteilhaft kann sie in den Bereichen Anwendung finden, bei denen zum einen eine flüssigkeits- und/oder gasdichte Abgrenzung zu einer Gerätschaft oder einem Gewebe gewünscht wird und zum anderen eine Ableitung von Flüssigkeiten oder Gasen entlang der Folie vorteilhaft ist. Je nach Bedarf kann hierbei sowohl die eine als auch die andere Folienseite vorteilhaft in der Nutzung sein. Besonders vorteilhaft ist hierbei, daß durch die dünnwandige Folie keine wesentliche Zunahme des mit der Folie beschlagenen Geräts vorgenommen wird.

Wie vorstehend im einzelnen erläutert, lassen sich sog. gecoverte selbstexpandierende Stents, die zur Leckagenbehandlung im Gastrointestinaltrakt angewendet werden, mit einer offenporigen Folie ausrüsten. Wenn hier die Folie als Coverfolie auf dem Stent benutzt wird (offenporige Seite zum Gewebe, glatte geschlossene Seite zum Stent), ist gleichzeitig die übliche Stentüberbrückung mit einer Vakuumsogausübung auf das anliegende Gewebe möglich.

Besonders vorteilhaft wäre eine solche Nutzung bei einer Lumeninkongruenz von den zu überbrückenden Darmlumina oder im Bronchialsystem. Es können in gleicher Weise Tuben, Overtubes, Sonden, Endoskope, die intrakorporal eingeführt werden, von einer offenporigen Hülle umhüllt werden. Wenn die geschlossene Seite geräteseitig liegt, ist die gleichzeitige Ausübung des Vakuums auf das Gewebe und Ausübung der Vakuumtherapie möglich. Wenn die Folie mit der offenporigen Seite an das medizinische Gerät gelegt wird kann das als Schutzumhüllung für das medizinische Gerät dienen. Durch den Sog schließt die Folie mit dem Gerät ab und trägt nicht wesentlich zur Erhöhung des Umfangs bei.

Vorteilhaft ist auch die Ausrüstung von Beatmungstuben und Narkosetuben möglich. Hier wird bislang eine Abdichtung des Tubus gegenüber der Trachea mit einem Ballon vorgenommen. Wenn hier der Tubus mit einer einseitig offenporigen Folie ausgerüstet ist, kann die Abdichtung über das Vakuum vorgenommen werden. Bei allen medizinischen Instrumenten, bei denen eine Fixierung durch eine Ballonexpansion vorgenommen wird, kann eine Fixierung und Abdichtung auch durch Vakuumsog erfolgen. Ein weiteres Beispiel einer Geräteausrüstung ist die Verwendung der Folie bei der Vakuumendoskopie als endoskopisches Untersuchungsverfahren des Dünndarms. In Analogie zur Single- oder Doppelballonendoskopie kann die Fixierung des Overtubes und des Endoskops durch Sog erfolgen.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert.

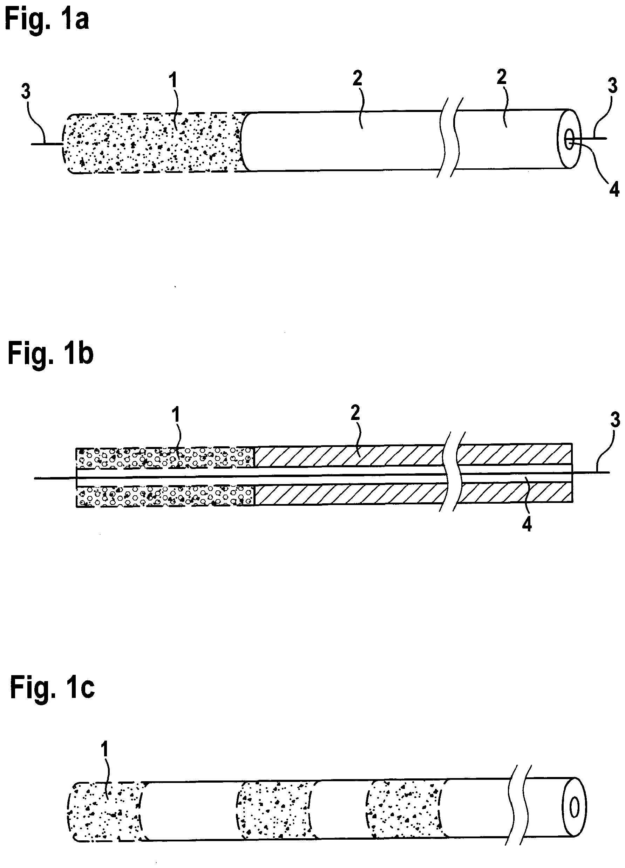

**Fig. 1a** ist eine Darstellung einer erfindungsgemäßen Unterdruckbehandlungsanordnung in Form eines offenporigen Drainageschlauchs. Das offenporige Fluidsammelsegment 1 bzw. Kontaktelement 1 befindet sich am distalen Ende des schlauchförmigen Fluidkommunikationselements 2. Ein Führungsdraht 3 ist in einen Kanal 4 eingeführt.
**Fig. 1b** ist eine Querschnittsdarstellung von Fig. 1a. Fluidsammelsegment 1 und Fluidkommunikationselement 2 gehen stufenlos ineinander über. In beide ist über einen Kanal 4 der Führungsdraht 3 eingeführt. Der Kanal 4 ist fluidleitend mit dem Fluidsammelsegment 1 bzw. Kontaktelement 1 verbunden.
**Fig. 1c** ist eine Darstellung eines offenporigen Drainageschlauchs mit mehreren offenporigen Fluidsammelsegmenten 1 bzw. Kontaktelementen 1.

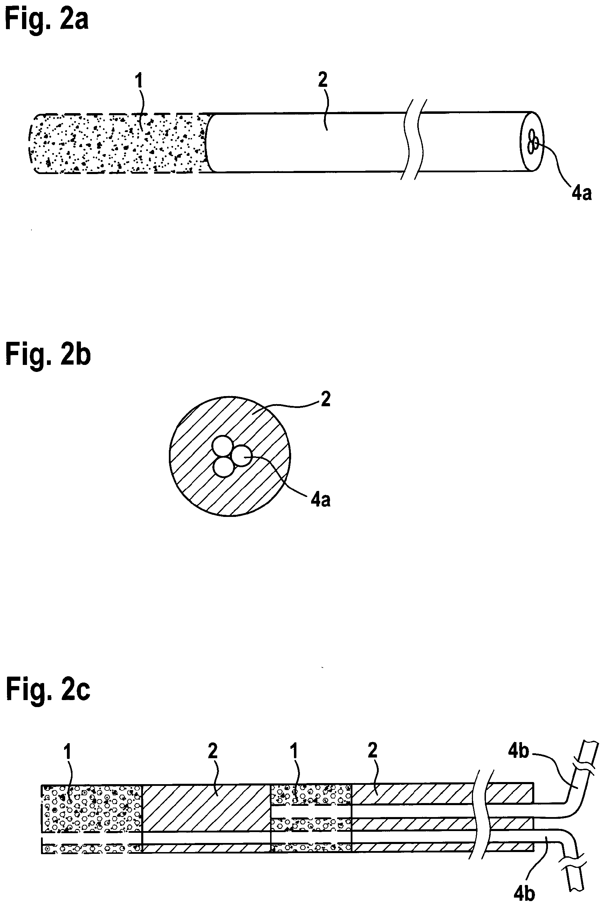

**Fig. 2a** ist eine Darstellung einer erfindungsgemäßen Anordnung in Form eines offenporigen Drainageschlauchs. Das offenporige Fluidsammelsegment 1 bzw. Kontaktelement 1 befindet sich am distalen Ende des schlauchförmigen Fluidkommunikationselements 2. Im Fluidkommunikationselement 2 ist ein dreilumiger Kanal 4a angeordnet.
**Fig. 2b** ist eine Querschnittsdarstellung von Fig. 2a in Höhe des Fluidkommunikationselements 2. Zentral ist ein dreilumiger Kanal 4a vorhanden.
**Fig. 2c** ist eine Längsschnittdarstellung eines offenporigen Drainageschlauchs mit zwei Fluidsammelsegmenten 1. Jedes Fluidsammelsegment 1 bzw. Kontaktelement 1 ist mit einem Kanal 4b, welcher das Fluidkommunikationselement 2 durchzieht, fluidleitend verbunden.

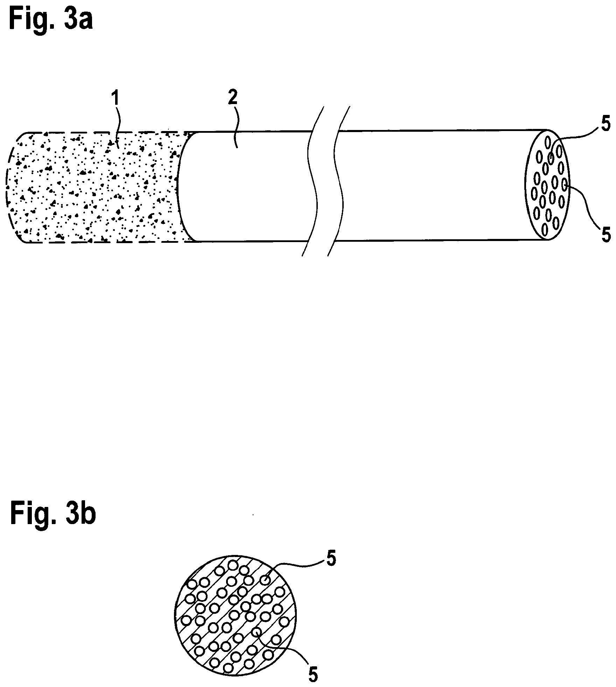

**Fig. 3a** ist eine Darstellung eines offenporigen Drainageschlauchs. Das offenporige Fluidsammelsegment 1 bzw. Kontaktelement 1 befindet sich am distalen Ende des schlauchförmigen Fluidkommunikationselements 2. In dem Fluidkommunikationselement 2 befindet sich eine Vielzahl kleinlumiger Kanäle 5, die sich fluidleitend bis zum Fluidsammelsegment 1 erstrecken.
**Fig. 3b** ist eine Querschnittsdarstellung von Fig. 3a in Höhe des Fluidkommunikationselements 2, welches mit einer Vielzahl von Kanälen 5 versehen ist.

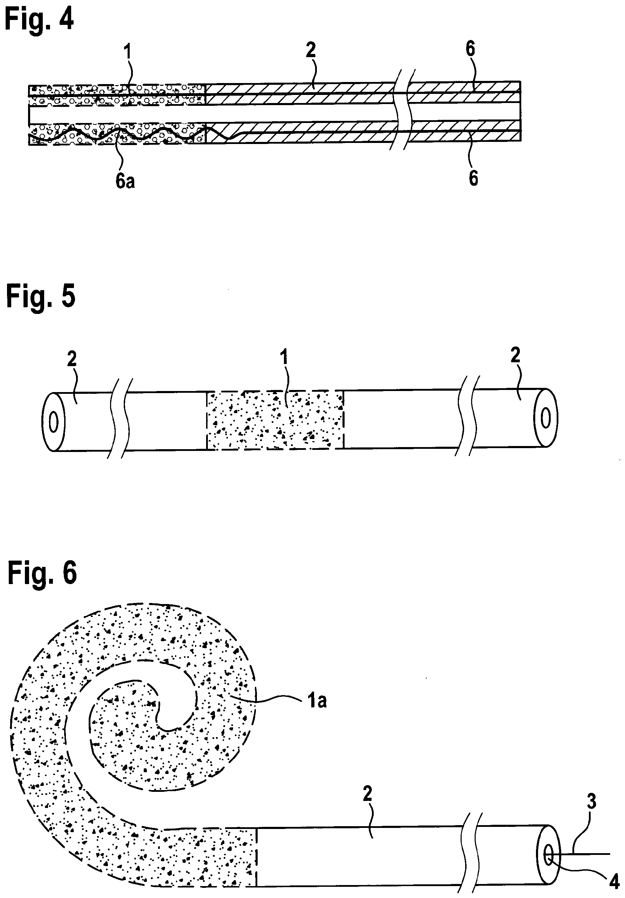

**Fig. 4** ist eine Längsschnittdarstellung einer erfindungsgemäßen Anordnung in Form eines offenporigen Drainageschlauchs. Das offenporige Fluidsammelsegment 1 bzw. Kontaktelement 1 befindet sich am distalen Ende des schlauchförmigen Fluidkommunikationselements 2. In der Wand des Schlauchs befindet sich zur Erhöhung der Zugfestigkeit der Wand ein drahtförmiger Faden 6. Dieser kann auch geschlängelt und in Windungen 6a verlaufen.
**Fig. 5** ist eine Darstellung einer erfindungsgemäßen Anordnung in Form eines offenporigen Drainageschlauchs. Das offenporige Fluidsammelsegment 1 bzw. Kontaktelement 1 befindet sich in der Mitte eines schlauchförmigen Fluidkommunikationselements 2.
**Fig. 6** ist eine Darstellung einer erfindungsgemäßen Anordnung in Form eines offenporigen Drainageschlauchs. Ein spiralförmig gekrümmtes offenporiges Fluidsammelsegment 1a befindet sich am distalen Ende des schlauchförmigen Fluidkommunikationselements 2. In einen fluidleitenden Kanal 4 ist ein Führungsdraht 3 eingeführt.

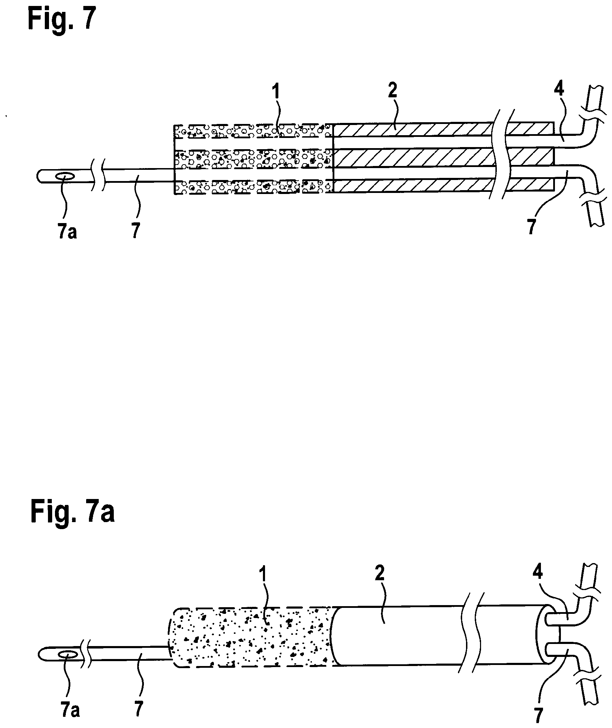

**Fig. 7** ist eine Längsschnittdarstellung einer erfindungsgemäßen Anordnung in Form eines offenporigen Drainageschlauchs. Das offenporige Fluidsammelsegment 1 bzw. Kontaktelement 1 befindet sich am distalen Ende des schlauchförmigen Fluidkommunikationselements 2. Ein Schlauch 7 ist hindurchgeführt, welcher am Ende eine Perforationsöffnung 7a besitzt. Dieser kann als Ernährungssonde eingesetzt werden. Ein Kanal 4 ist fluidleitend mit dem Fluidsammelsegment 1 verbunden.
**Fig. 7a** ist eine weitere Darstellung von Fig. 7. Das offenporige Fluidsammelsegment 1 bzw. Kontaktelement 1 befindet sich am distalen Ende des schlauchförmigen Fluidkommunikationselements 2. Ein Schlauch 7 ist hindurchgeführt, welcher am Ende eine Perforationsöffnung 7a besitzt. Dieser kann als Ernährungssonde eingesetzt werden. Ein Kanal 4 ist fluidleitend mit dem Fluidsammelsegment 1 verbunden.

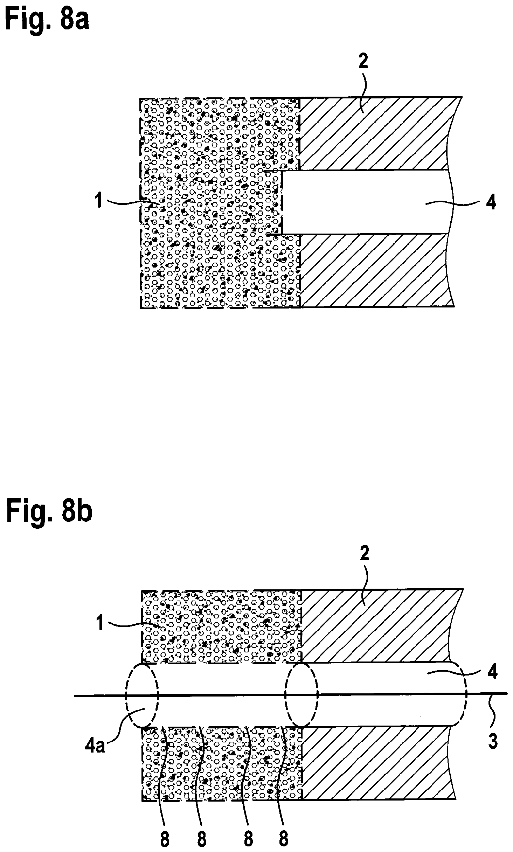

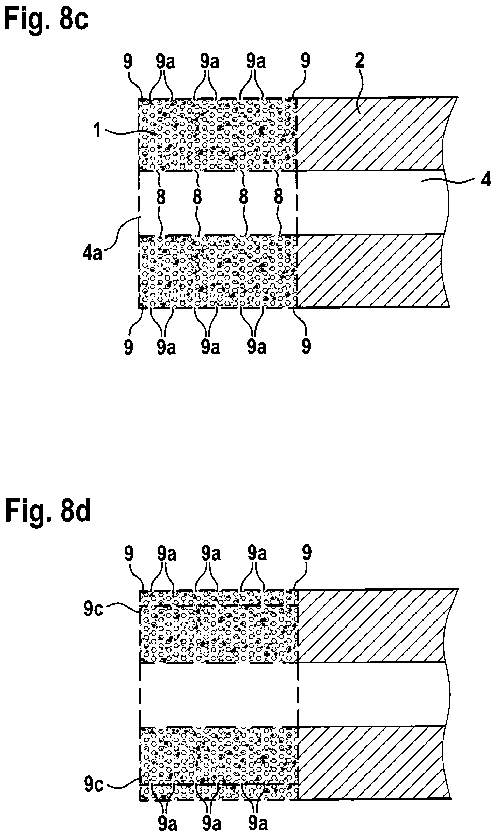

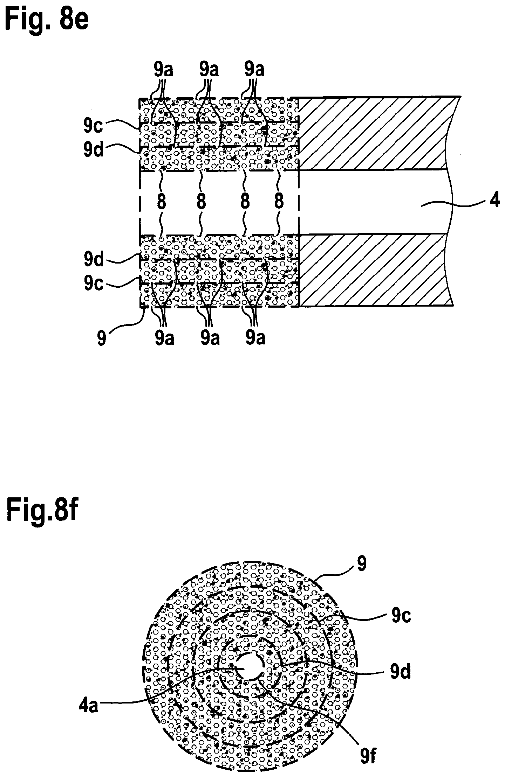

**Fig. 8** ist eine Darstellung des offenporigen Drainageschlauchs. Es werden verschiedene Varianten der Übergangsstellen vom Fluidsammelsegment 1 zum Fluidkommunikationselement 2 dargestellt.
**Fig. 8a** ist eine Längsschnittdarstellung des Übergangs vom Fluidsammelsegment 1 zum Fluidkommunikationselement 2. Das offenporige Fluidsammelsegment 1 bzw. Kontaktelement 1 ist stufenlos mit dem Fluidkommunikationselement 2 verbunden. Im Fluidkommunikationselement 2 ist ein fluidleitender Kanal 4 vorgesehen.
**Fig. 8b** ist eine Längsschnittdarstellung des Übergangs vom Fluidsammelsegment 1 zum Fluidkommunikationselement 2. Das offenporige Fluidsammelsegment 1 bzw. Kontaktelement 1 ist stufenlos mit dem Fluidkommunikationselement 2 verbunden. Im Fluidkommunikationselement 2 befindet sich ein fluidleitender Kanal 4, der als unterdruckstabiler Schlauch in dem Fluidsammelsegment 1 weitergeführt ist und mit seitlichen Perforationsöffnungen 8 fluidleitend mit dem Fluidsammelsegment 1 verbunden ist. In den Kanal 4 ist ein Führungsdraht 3 eingeführt.
**Fig. 8c** ist eine Längsschnittdarstellung des Übergangs vom Fluidsammelsegment 1 zum Fluidkommunikationselement 2. Das offenporige Fluidsammelsegment 1 bzw. Kontaktelement 1 ist stufenlos mit dem Fluidkommunikationselement 2 verbunden. Im Fluidkommunikationselement 2 befindet sich ein fluidleitender Kanal 4, der als unterdruckstabiler Schlauch in dem Fluidsammelsegment 1 weitergeführt ist und mit seitlichen Perforationsöffnungen 8 fluidleitend mit dem Fluidsammelsegment 1 verbunden ist. Das Fluidsammelsegment 1 ist mit einer Folie 9 mit fluidleitenden Perforationsöffnungen 9a überzogen. Die Folie 9 geht stufenlos in das Fluidkommunikationselement 2 über. Die Außenbeschichtung der Folie 9 soll zu einem verbesserten Gleitvermögen der Drainage führen, welches die Platzierung und Entfernung erleichtert.
**Fig. 8d** entspricht der Längsschnittdarstellung in Fig. 8c. Zusätzlich ist das offenporige Fluidsammelsegment 1 bzw. Kontaktelement 1 von einer weiteren Folie 9c mit fluidleitenden Perforationsöffnungen 9a ausgerüstet bzw. durchsetzt.
**Fig. 8e** entspricht der Längsschnittdarstellung in Fig. 8d. Zusätzlich ist das offenporige Fluidsammelsegment 1 bzw. Kontaktelement 1 von einer weiteren schlauchförmigen Folie 9d mit fluidleitenden Perforationsöffnungen 9a ausgerüstet bzw. durchsetzt. Durch die mehrlagige Folienkonstruktion wird die Zugfestigkeit erhöht. Durch die Konstruktion von offenporigen mehrlagigen Folien 9, 9c, 9d soll eine maximale Fluidleitung bei geringem Durchmesser der Drainage erreicht werden.
**Fig. 8f** entspricht der Querschnittsdarstellung einer mit vier schlauchförmigen Folienlagen 9, 9c, 9d, 9f ausgerüsteten Drainage mit zentralem fluidleitendem Kanal 4a.

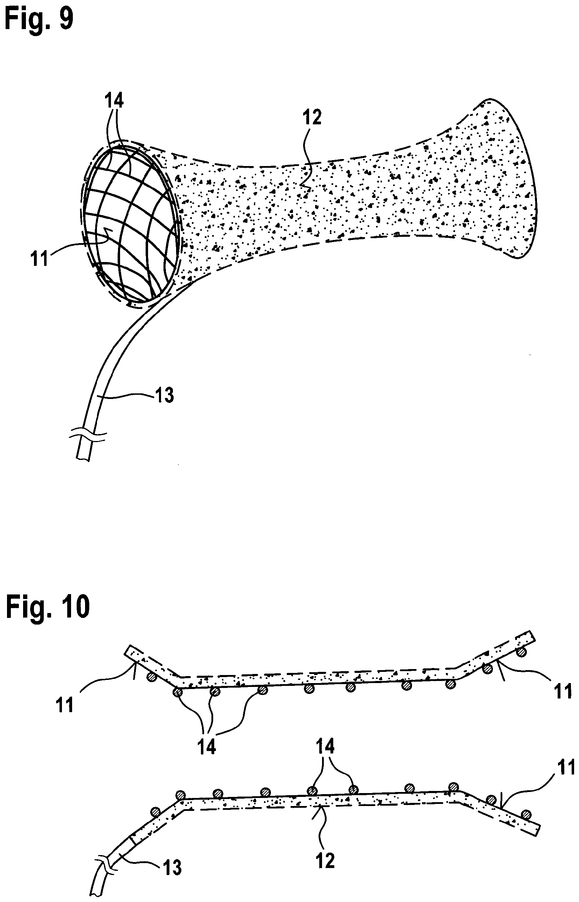

**Fig. 9** ist eine Aufsichtsdarstellung eines selbstexpandierenden Metall- oder Kunststoffgitterstents, welcher aus einem selbstexpandierenden Metall- oder Kunststoffdrahtgitter 14 besteht. Der Stent ist vollständig mit einer einseitig offenporigen Folie 9 bzw. mit einem Kontaktelement 1 ummantelt, deren Außenseite 12 eine offenporige Struktur hat und deren Innenseite 11, die nicht offenporig ist, an den Metall- oder Kunststoffgitterdrähten 14 anliegt. Die äußere offenporige Oberfläche ist mit einem Drainageschlauch 13 fluidleitend verbunden. Beide Enden sind trichterförmig erweitert.
**Fig. 10** ist eine Längsschnittdarstellung von Fig. 1. Die innen liegende Oberflächenseite 11 der Folie ist nicht offenporig und liegt an den Metall- oder Kunststoffgitterdrähten 14 an. Die offenporige Oberflächenseite 12 der Folie ist außen liegend und mit einer schlauchförmigen Drainage 13 fluidleitend verbunden. Beide Enden sind trichterförmig erweitert.

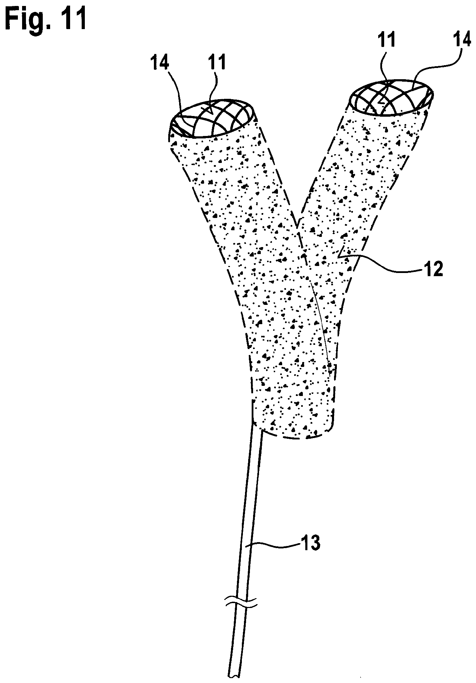

**Fig. 11** ist eine Aufsichtsdarstellung eines selbstexpandierenden γ-förmigen Metall-oder Kunststoffgitterstents, welcher aus einem selbstexpandierenden Metall- oder Kunststoffdrahtgitter 14 besteht. Der Stent ist vollständig mit einer einseitig offenporigen Folie ummantelt, deren Außenseite 12 eine offenporige Struktur hat und deren Innenseite 11, die nicht offenporig ist, an den Metall- oder Kunststoffgitterdrähten 14 anliegt. Die äußere offenporige Oberfläche 12 ist mit einem Drainageschlauch 13 fluidleitend verbunden. Beispielhaft soll dieses Ausführungssbeispiel eines γ-förmigen Stents die Möglichkeit der Anwendung im tracheobronchialen System abbilden.

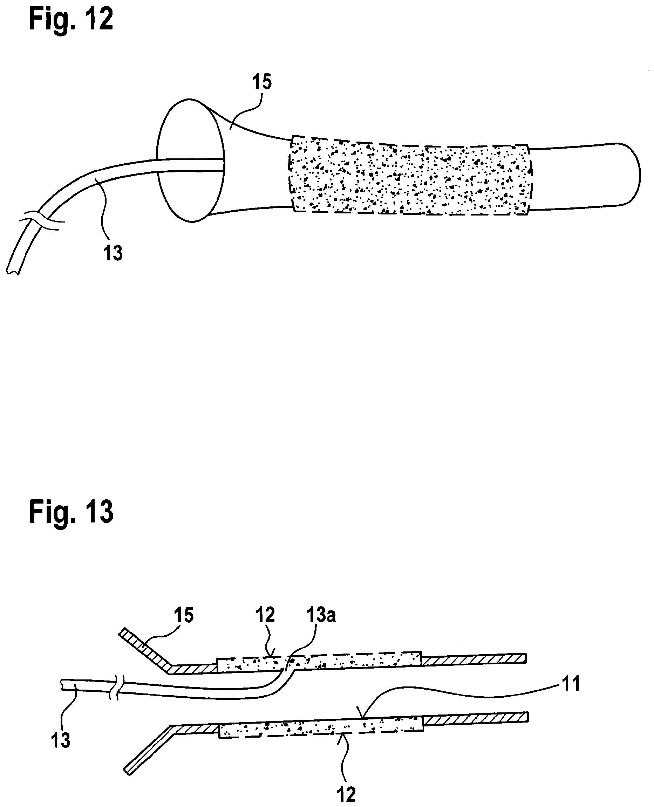

**Fig. 12** ist eine Aufsichtsdarstellung eines röhrenförmigen Tubus, dessen Wand im mittleren Abschnitt des Tubus circulär eine einseitig offenporige Struktur hat. Mit 12 ist die außen sichtbare offenporige Struktur der Wand gekennzeichnet. Diese ist mit einer schlauchförmigen Drainageleitung 13, die von der Innenseite des Tubus herangeführt wird, fluidleitend verbunden. Ein Ende ist trichterförmig erweitert.
**Fig. 13** ist eine Längsschnittdarstellung von Fig. 4. Die Wand des Tubus ist einseitig offen konstruiert. Die Innenseite 11 der Wand ist nicht offenporig, die Außenwand 12 ist im mittleren Teil des Tubus offenporig. Von innen wird über eine Perforationsöffnung 13a eine Drainageleitung 13 fluidleitend an die Außenseite herangeführt. Ein Ende ist trichterförmig erweitert.

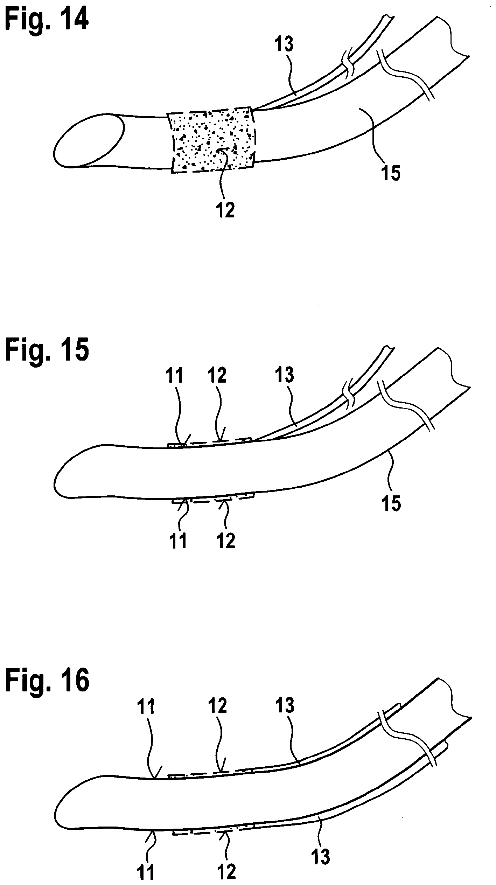

**Fig. 14** ist eine Aufsichtsdarstellung einer Sonderform eines Tubus. Es handelt sich um einen Intubationstubus. Das Tubusrohr 15 wird im distalen Abschnitt von einer offenporigen Folie umhüllt. Ein Schlauch 13 führt fluidleitend zu der offenporigen Oberfläche 12.
**Fig. 15** ist eine Längsschnittdarstellung von Fig. 6. Das Tubusrohr 15 ist am distalen Abschnitt von einer offenporigen Folie umhüllt. 12 ist die offenporige außen liegende Oberfläche. Die nicht offenporige Oberfläche 11 liegt dem Tubusrohr 15 an. Es besteht eine fluidleitende Verbindung mit einem Schlauch 13.
**Fig. 16** ist eine Längsschnittdarstellung eines Tubus, bei dem die Wand des Tubus selbst außen offenporig 12 und nach innen nicht offenporig 11 ist. In der Wand einliegend sind die schlauchförmigen Drainageleitungen 13, die mit der offenporigen Oberfläche fluidleitend verbunden sind.

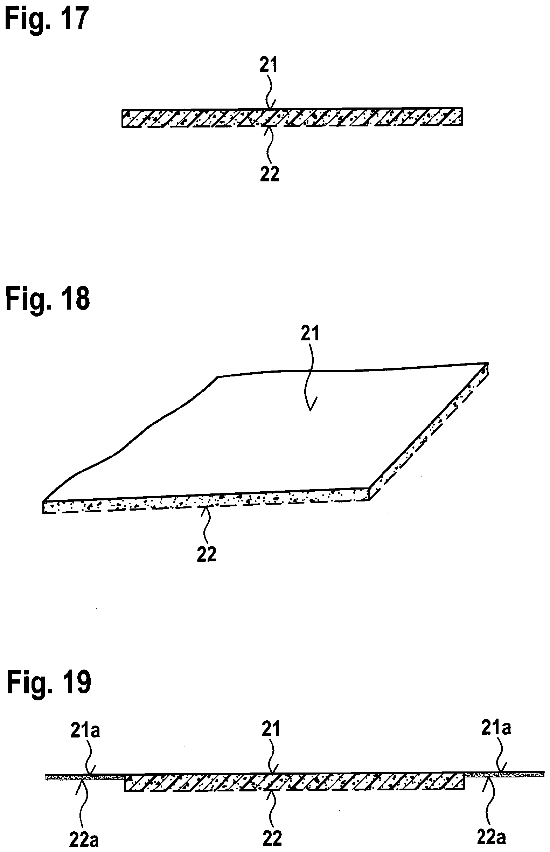

**Fig. 17** ist eine Querschnittsdarstellung einer einseitig offenporigen Folie mit einer nicht offenporigen Oberflächenseite 21 und einer offenporigen Oberflächenseite 22.
**Fig. 18** ist eine Aufsichtsdarstellung einer einseitig offenporigen Folie mit einer nicht offenporigen Oberflächenseite 21 und einer offenporigen Oberflächenseite 22. Die Folie ist rechteckig zugeschnitten.
**Fig. 19** ist eine Querschnittsdarstellung einer einseitig offenporigen Folie mit einer nicht offenporigen Oberflächenseite 21 und einer offenporigen Oberflächenseite 22. Im Randbereich der Folie sind sowohl die offenporige Oberflächenseite 21a als auch die offenporige Oberflächenseite 22a nicht offenporig. Der Randbereich 21a und/oder 22a kann mit einem Klebstoff beschlagen sein, so daß die Folie im Randbereich 21a, 22a gas- und luftdicht abschließt, wenn sie aufgeklebt und/oder miteinander verklebt wird.

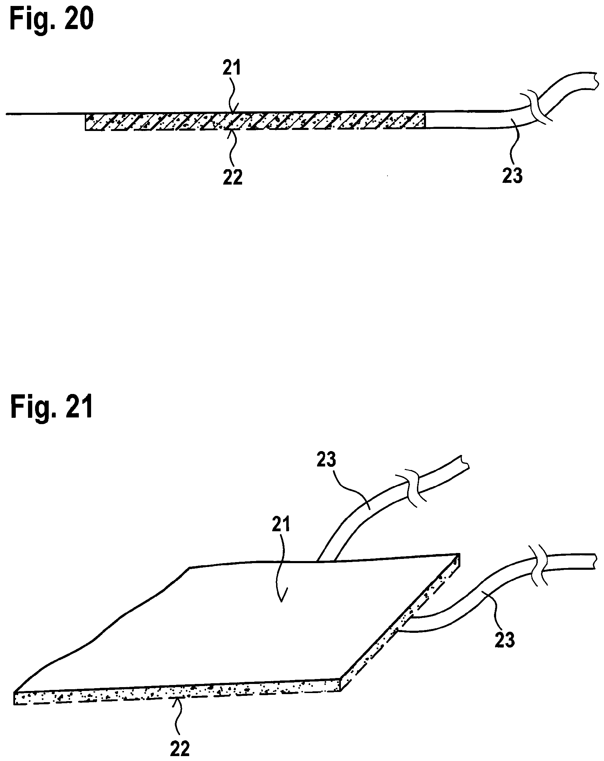

**Fig. 20** ist eine Querschnittsdarstellung einer einseitig offenporigen Folie mit einer nicht offenporigen Oberflächenseite 21 und einer offenporigen Oberflächenseite 22. Mit der offenporigen Oberflächenseite 22 ist fluidleitend ein schlauchförmiges Fluidkommunikationsmittel 23 verbunden, welches von außen an die offenporige Oberflächenseite 22 herangeführt ist und mit dieser fluidleitend verbunden ist.
**Fig. 21** ist eine Aufsichtsdarstellung einer einseitig offenporigen Folie mit einer nicht offenporigen Oberflächenseite 21 und einer offenporigen Oberflächenseite 22. Mit der offenporigen Oberflächenseite 22 sind fluidleitend zwei schlauchförmige Fluidkommunikationsmittel 23 verbunden, welche von außen an die offenporige Oberflächenseite 22 herangeführt werden und mit dieser fluidleitend verbunden sind.

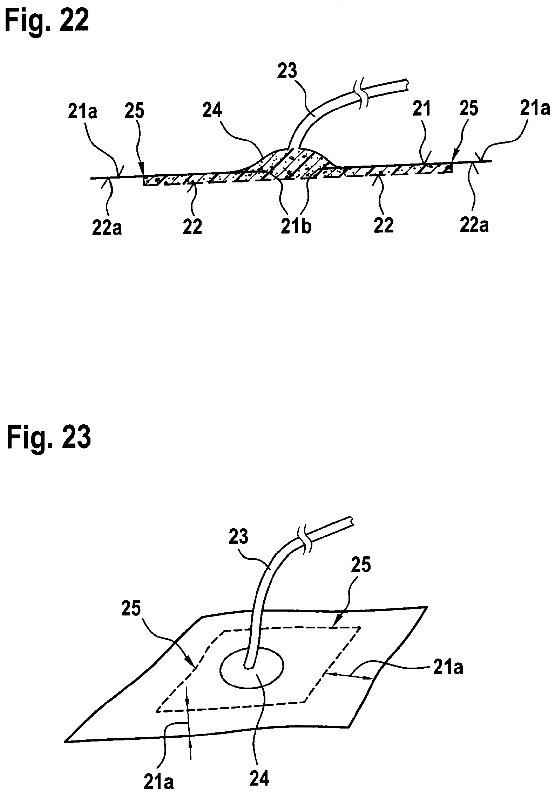

**Fig. 22** ist eine Querschnittsdarstellung einer einseitig offenporigen Folie mit einer nicht offenporigen Oberflächenseite 21 und einer offenporigen Oberflächenseite 22. Mit einer Pelotte 24 wird das schlauchartige Fluidkommunikationsmittel 23 über eine Öffnung 21b der nicht offenporigen Oberflächenseite 21 mit der offenporigen Oberflächenseite 22 fluidleitend verbunden. 25 kennzeichnet den Übergang, bei dem beide Oberflächenseiten im Randbereich in eine nicht offenporige Oberfläche (21a und 22a) übergehen.
**Fig. 23** ist eine Aufsichtsdarstellung der Fig. 22. Die fluidleitende Pelotte 24 ist zentral auf einer rechteckigen Folie befestigt. Die Folie ist im Randbereich 22a von beiden Seiten nicht offenporig. Der Übergang zur offenporigen (hier nicht sichtbaren) Oberfläche ist mit 25 gekennzeichnet.

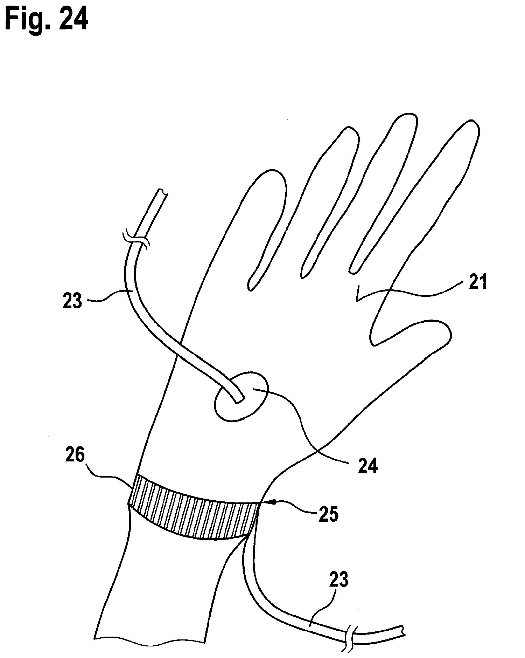

**Fig. 24** ist die beispielhafte Darstellung einer an eine Bekleidungsform angepaßten Folie, hier eine Handschuhform. Außen ist die nicht offenporige Folienseite 21, der offenporigen (nicht sichtbaren innen liegenden) Folienseite werden schlauchförmige Fluidkommunikationsmittel 23 zugeleitet, davon einer über eine Pelotte 24. Der Abschluß 26 des Handschuhs ist mit der Haut verklebt, der innere nicht sichtbare Übergang zur offenporigen Seite des Randbereichs ist mit 25 gekennzeichnet.

### BEZUGSZEICHENLISTE

- 1, 1a: Fluidsammelsegment / Kontaktelement
- 2: Fluidkommunikationselement
- 3: Führungsdraht
- 4, 4a, 4b, 5: Kanal
- 6: drahtförmiger Faden
- 6a: Windungen
- 7: Schlauch
- 7a, 8, 9a, 13a: Perforationsöffnungen
- 9, 9c, 9d, 9f: Folie
- 11: Innenseite / innen liegende Oberflächenseite der Folie
- 12: Außenseite / außen liegende Oberflächenseite der Folie / Außenwand
- 13: Drainageschlauch / Drainageleitung
- 14: Metall- oder Kunststoffgitterdrähte / Metall- oder Kunststoffdrahtgitter
- 15: Tubusrohr
- 21: Oberflächenseite der Folie (nicht offenporig)
- 22: Oberflächenseite der Folie (offenporig)
- 21a, 22a: Randbereich
- 21b: Öffnung
- 23: Fluidkommunikationsmittel
- 24: Pelotte
- 25: Übergang
- 26: Abschluß (des Handschuhs)

## Patentansprüche

1. Unterdruckbehandlungsanordnung mit mindestens einem als Fluidsammelelement zum Sammeln von Flüssigkeiten oder Gasen ausgeführten offenporigen Kontaktelement (1), über das ein Unterdruck und/oder Sog in einer Körperhöhle erzeugt werden kann, wobei das offenporige Kontaktelement (1) zumindest abschnittweise schlauchartig mit einer eine Schlauchachse zumindest teilweise umlaufenden äußeren und/oder inneren Begrenzungsfläche ausgeführt ist, wobei das Fluidsammelelement fluidleitend derart mit einem kanalförmigen Lumen eines Drainageschlauchs (13) verbunden ist, dass eine schlauchförmige Drainage gebildet ist, bei der das Fluidsammelelement als Teil einer Schlauchwand und der ein Fluidkommunikationselement (2) bildende Drainageschlauch (13) in sich konstruktiv vereinigt sind,
**dadurch gekennzeichnet, dass** das Fluidsammelelement radial nicht über den Drainageschlauch hinaussteht und das Fluidsammelelement und der Drainageschlauch von gleichem Außendurchmesser sind und stufenlos ineinander übergehen.

2. Unterdruckbehandlungsanordnung nach Anspruch 1, bei welcher sich mindestens ein offenporiges Fluidsammelelement der Schlauchwand abschnittweise zwischen proximalem und distalem Ende des Drainageschlauchs (13) befindet und/oder bei dem sich ein offenporiges Fluidsammelelement der Schlauchwand am distalen Ende des Drainageschlauchs (13) befindet.

3. Unterdruckbehandlungsanordnung nach einem der vorhergehenden Ansprüche, bei welcher die offenporigen Fluidsammelelemente der Schlauchwand eine Länge von wenigen mm bis zu 30 cm haben und bei dem die offenporigen Fluidsammelelemente eine Porengröße von 200 µm bis zu 1000 µm aufweisen und/oder bei dem die Offenporigkeit der Schlauchwand durch die konstruktive Einarbeitung eines offenporigen Schwammkörpers und/oder einer offenporigen Folie in der Schlauchwand erzielt wird, wobei das Fluidsammelelement aus Polyurethan und/oder Polyvinyl und/oder Polyethylen besteht.

4. Unterdruckbehandlungsanordnung nach einem der vorhergehenden Ansprüche, bei welcher das kanalförmige Fluidkommunikationselement (2) mindestens einen zentralen Kanal (4) und/oder mehrere Kanäle (4, 4a, 4b, 5) aufweist und/oder bei dem sich Kanäle (4, 4a, 4b, 5) in der Wandung des Drainageschlauchs (13) befinden, welche fluidleitend mit den offenporigen Fluidsammelelementen der Schlauchwand verbunden sind.

5. Unterdruckbehandlungsanordnung nach einem der vorhergehenden Ansprüche, bei der die fluidleitenden Kanäle (4, 4a, 4b, 5) gleichlumig sind oder verschiedene Lumina besitzen und die Kanäle (4, 4a, 4b, 5) untereinander fluidleitend sind und/oder bei der einzelne Kanäle (4, 4a, 4b, 5) fluidleitend mit einzelnen und/oder mehreren Fluidsammelsegmenten (1) verbunden sind.

6. Unterdruckbehandlungsanordnung nach einem der vorhergehenden Ansprüche, bei welcher fluidleitende Kanäle (4, 4a, 4b, 5) über die eigentliche Drainage einige cm oder dm hinausragen, so dass sie als Ernährungssonde genutzt werden können.

7. Unterdruckbehandlungsanordnung nach einem der vorhergehenden Ansprüche, bei welcher ein Führungsdraht (3) in einen Kanal (4) eingeführt werden kann und/oder bei dem der Drainageschlauch (13) an seinem distalen Ende mit einer Krümmung (Pigtail) versehen ist.

8. Unterdruckbehandlungsanordnung nach einem der vorhergehenden Ansprüche, welche eine Länge von 80 cm bis 250 cm und einem Außendurchmesser von 2 mm bis 20 mm aufweist, wobei kleinkalibrige Drainagen über den Arbeitskanal eines Endoskops eingeführt werden können.

9. Unterdruckbehandlungsanordnung nach einem der vorhergehenden Ansprüche, bei der zur Erhöhung der Zugfestigkeit des Kontaktelements (1) und/oder Fluidkommunikationselements (2) Fäden oder Drähte eingearbeitet sind und bei der eine Zugfestigkeit bis zu 200 N besteht und/oder bei der die Drainage röntgendicht ist und/oder bei der am distalen Ende der Drainage eine Vorrichtung zum Fassen der Drainage mit endoskopischen Instrumenten vorgesehen ist.

10. Unterdruckbehandlungsanordnung nach einem der vorhergehenden Ansprüche, welche über Verbindungselemente fluidleitend mit einem vakuumerzeugenden System, insbes. mit einer elektronischen Vakuumpumpe, verbunden wird, wobei ein Vakuum von 40 mmHg bis 200 mmHg zur Anwendung kommt.

11. Unterdruckbehandtungsanordnung nach einem der vorhergehenden Ansprüche, mit einem röhrenförmigen Hohlkörper für medizinische Anwendungen im menschlichen oder tierischen Körper, dessen Außenseite aus dem Kontaktelement (1) besteht, bei der das Kontaktelement (1) aus einer für Gase und Flüssigkeiten nicht permeablen Membran besteht, deren nach außen liegende Seite eine offenporige Oberfläche (12) aufweist, entlang dieser Oberflächenseite (12) Flüssigkeiten und/oder Gase fließen können, und deren nach innen liegende Seite eine nicht offenporige Oberfläche (11) aufweist.

12. Unterdruckbehandlungsanordnung nach Anspruch 11, bei der der Hohlkörper aus einem mit einer einseitig offenporigen Folie wenigstens teilweise ummantelten selbstexpandierenden Metall- und/oder Kunststoffgitterstent oder aus einem röhrenförmigen Tubus (15) besteht.

13. Unterdruckbehandlungsanordnung nach Anspruch 11 oder 12, bei der die offenporige Oberflächenseite (22) fluidleitend mit mindestens einem Fluidkommunikationselement (2) verbunden ist, welches fluidleitend mit einem unterdruckerzeugenden System verbunden werden kann und bei dem das Fluidkommunikationselement (2) aus einem Schlauch (7) besteht, der vorzugsweise von dem Hohlköper lösbar ist.

14. Unterdruckbehandlungsanordnung nach einem der Ansprüche 11 bis 13, bei der wenigstens ein Fluidkommunikationselement (2) durch eine die nicht offenporige Oberflächenseite (21) durchsetzende Öffnung (21 b) fluidleitend mit der offenporigen Oberflächenseite (22) verbunden ist.

15. Unterdruckbehandlungsanordnung nach einem der Ansprüche 11 bis 14, bei der die offenporige Oberflächenstruktur (22) gitter-, netz-, blasen-, noppen-, finger- oder kanalförmig ist.

16. Unterdruckbehandlungsanordnung nach einem der Ansprüche 11 bis 15, bei der der Hohlkörper aus einem röhrenförmigen Tubus (15) besteht, dessen Wandung mindestens teilweise einseitig offenporig konstruiert ist, wobei die Außenseite des Tubus (15) als offenporige Oberfläche (22) konstruiert ist und die offenporigen Eigenschaften der offenporigen Folie hat, und die Innenseite nicht offenporig ist.

17. Unterdruckbehandlungsanordnung nach einem der Ansprüche 11 bis 16, bei der der Hohlkörper an einem Ende oder an beiden Enden eine trichterförmige Aufweitung hat.

18. Unterdruckbehandlungsanordnung nach einem der Ansprüche 11 bis 17, bei der es sich bei dem Tubus (15) um einen ein- oder doppellumigen Intubationstubus handelt.

19. Unterdruckbehandlungsanordnung nach einem der Ansprüche 11 bis 18, bei der die offenporige Oberflächenseite (22) der Folie mit wenigstens einem Fluidkommunikationselement (2) mit einem vakuumerzeugenden System verbunden ist, welches ausgestaltet ist, um einen Unterdruck von bis zu 200 mmHg an die offenporige Folienoberfläche (22) anzulegen.

20. Unterdruckbehandlungsanordnung nach einem der Ansprüche 11 bis 19, bei der die Offenporigkeit der einen Folienseite durch die Beaufschlagung mit einem offenporigen Fluidsammelelement herbeigeführt wird und/oder bei dem das Fluidsammelelement aus einem offenporigen Polyurethanschaum besteht und bei dem die Porengröße der offenporigen Oberfläche zwischen 200 µm und 1000 µm beträgt.

## Claims

1. A negative pressure treatment arrangement comprising at least one open-cell contact element (1) designed as a fluid-collecting element for collecting fluids or gases, by means of which a negative pressure and/or suction can be generated in a body cavity, wherein the open-cell contact element (1) is designed, in sections at least, in the form of a tube with a tubular axis, said open-cell contact element having, at least in part, an outer and/or inner boundary area, wherein the fluid-collecting element is fluid-conductively connected to a channel-shaped lumen of a drainage tube (13) in such a way that a tubular drain is formed in which the open-cell fluid-collecting element as part of a tube wall and the drainage tube (13) which forms a fluid-communicating element (2) are structurally interconnected,
**characterised in that** the fluid-collecting element does not protrude radially over the drainage tube and the fluid-collecting element and the drainage tube have the same outer diameter and merge continuously into each other.

2. The negative pressure treatment arrangement according to claim 1, in which at least one open-cell fluid-collecting element of the tube wall is located in sections between the proximal and the distal end of the drainage tube (13) and/or in which an open-cell fluid-collecting element of the tube wall is located at the distal end of the drainage tube (13).

3. The negative pressure treatment arrangement according to one of the preceding claims, in which the open-cell fluid-collecting elements of the tube wall have a length of a few millimetres up to 30 cm and in which the open-cell fluid-collecting elements have a cell size of 200 µm up to 1000 µm and/or in which the open-cell structure of the tube wall is achieved by the structural incorporation of an open-cell sponge element and/or an open-cell film in the tube wall, the fluid-collecting element consisting of polyurethane and/or polyvinyl and/or polyethylene.

4. The negative pressure treatment arrangement according to one of the preceding claims, in which the channel-shaped fluid-communicating element (2) has at least one central channel (4) and/or a plurality of channels (4, 4a, 4b, 5) and/or in which the channels (4, 4a, 4b, 5) are located in the wall of the drainage tube (13), with said channels being fluid-conductively connected to the open-cell fluid-collecting elements of the tube wall.

5. The negative pressure treatment arrangement according to one of the preceding claims, in which the fluid-conductive channels (4, 4a, 4b, 5) are equiluminous or have different lumens and the channels (4, 4a, 4b, 5) are mutually fluid-conductive and/or in which the individual channels (4, 4a, 4b, 5) are fluid-conductively connected to individual and/or a plurality of fluid-collecting segments (1).

6. The negative pressure treatment arrangement according to one of the preceding claims, in which fluid-conductive channels (4, 4a, 4b, 5) extend a few centimetres or decimetres beyond the drainage perse in such a way that they can be used as a feeding probe.

7. The negative pressure treatment arrangement according to one of the preceding claims, in which a guide wire (3) can be introduced into a channel (4) and/or in which the drainage tube (13) is provided with a curvature (pigtail) at its distal end.

8. The negative pressure treatment arrangement according to one of the preceding claims which has a length of 80 cm to 250 cm and an outside diameter of 2 mm to 20 mm, wherein small-calibre drains can be introduced via the working channel of an endoscope.

9. The negative pressure treatment arrangement according to one of the preceding claims, in which, in order to increase the tensile strength of the contact element (1) and/or fluid-communicating element (2), threads or wires are incorporated and in which a tensile strength of up to 200 N exists and/or in which the drain is radiopaque and/or in which a device for grasping the drain using endoscopic instruments is provided at the distal end of the drain.

10. The negative pressure treatment arrangement according to one of the preceding claims, which is fluid-conductively connected, via connecting elements, to a vacuum-generating system, especially to an electronic vacuum pump, with a vacuum of 40 mmHg to 200 mmHg being applied.

11. The negative pressure treatment arrangement according to one of the preceding claims comprising, for medical applications in the human or animal body, a tubular hollow body, the outside of which consists of the contact element (1), in which the contact element (1) consists of a membrane that is non-permeable to gases and fluids, and the side of which that is located on the outside has an open-cell surface (12), fluids and/or gases being able to flow along this surface side (12), and the side of which that is located on the inside has an open cell-free surface (11).

12. The negative pressure treatment arrangement according to claim 11, in which the hollow body consists of a self-expanding metal and/or plastic mesh stent at least partly jacketed in a film that is open-cell on one side and/or of a tubular barrel (15).

13. The negative pressure treatment arrangement according to claim 11 or 12, in which the open-cell surface side (22) is fluid-conductively connected to at least one fluid-communicating element (2), which can be fluid-conductively connected to a negative pressure-generating system and in which the fluid-communicating element (2) consists of a tube (7), which is preferably detachable from the hollow body.

14. The negative pressure treatment arrangement according to one of claims 11 to 13, in which at least one fluid-communicating element (2) is fluid-conductively connected, by means of a port (21b) that traverses the open cell-free surface side (21), to the open-cell surface side (22).

15. The negative pressure treatment arrangement according to one of claims 11 to 14, in which the open-cell surface structure (22) is mesh-, net-, bubble-, nub-, finger- or channel-shaped.

16. The negative pressure treatment arrangement according to one of claims 11 to 15, in which the hollow body consists of a tubular barrel (15), the walls of which are at least partially designed so as to be open-cell on one side, wherein the outside of the barrel (15) is designed as an open-cell surface (22) and has the open-cell characteristics of the open-cell film, the inside being open cell-free.

17. The negative pressure treatment arrangement according to one of claims 11 to 16, in which one end or both ends of the hollow body is or are flared in a funnel formation.

18. The negative pressure treatment arrangement according to one of claims 11 to 17, in which the barrel (15) is a single- or dual-lumen intubation tube.

19. The negative pressure treatment arrangement according to one of claims 11 to 18, in which the open-cell surface side (22) of the film is connected, via at least one fluid-communicating element (2), to a vacuum-generating system which is configured to apply a negative pressure of up to 200 mmHg to the open-cell film surface (22).

20. The negative pressure treatment arrangement according to one of claims 11 to 19, in which the open-cell structure of one of the film sides is brought about by the application of an open-cell fluid-collecting element and/or in which the fluid-collecting element consists of an open-cell polyurethane foam and in which the cell size of the open-cell surface is between 200 µm and 1000 µm.

## Revendications

1. Dispositif de traitement par pression négative comportant au moins un élément de contact (1) poreux constituant un élément absorbeur de fluide destiné à absorber des fluides ou gaz et par le biais duquel une pression négative et/ou une aspiration peuvent être mises en œuvre dans une cavité corporelle, ledit élément de contact (1) poreux étant, au moins par sections, tubulaire et présentant une surface de délimitation extérieure et/ou intérieure entourant au moins partiellement un axe de tube, ledit élément absorbeur de fluide étant relié, de façon permettant de conduire des fluides, à une lumière en forme de canal d'un tube de drainage (13) de manière à former un drain tubulaire dans lequel l'élément absorbeur de fluide, constituant une partie d'une paroi du tube, et le tube de drainage (13), formant un élément de communication fluidique (2), sont structurellement unis,
**caractérisé en ce que** l'élément absorbeur de fluide ne dépasse pas radialement du tube de drainage et **en ce que** l'élément absorbeur de fluide et le tube de drainage sont d'un même diamètre extérieur et la transition de l'un à l'autre est totalement fluide.

2. Dispositif de traitement par pression négative selon la revendication 1, dans lequel au moins un élément poreux absorbeur de fluide de la paroi du tube se trouve, par sections, entre l'extrémité proximale et l'extrémité distale du tube de drainage (13) et/ou dans lequel un élément poreux absorbeur de fluide de la paroi du tube se trouve à l'extrémité distale du tube de drainage (13).

3. Dispositif de traitement par pression négative selon l'une des revendications précédentes, dans lequel les éléments poreux absorbeurs de fluide de la paroi du tube ont une longueur allant de quelques millimètres jusqu'à 30 cm et dans lequel les éléments poreux absorbeurs de fluide présentent une taille de pores allant de 200 µm à 1000 µm et/ou dans lequel la porosité de la paroi du tube est assurée par l'intégration structurelle d'un corps spongieux poreux et/ou d'une feuille poreuse dans la paroi du tube, ledit élément absorbeur de fluide se composant de polyuréthane et/ou de polyvinyle et/ou de polyéthylène.

4. Dispositif de traitement par pression négative selon l'une des revendications précédentes, dans lequel l'élément de communication fluidique (2) en forme de canal présente au moins un canal central (4) et/ou plusieurs canaux (4, 4a, 4b, 5) et/ou dans lequel des canaux (4, 4a, 4b, 5) se trouvent dans la paroi du tube de drainage (13), lesquels sont reliés, de façon permettant de conduire des fluides, aux éléments poreux absorbeurs de fluide de la paroi du tube.

5. Dispositif de traitement par pression négative selon l'une des revendications précédentes, dans lequel les canaux (4, 4a, 4b, 5) permettant de conduire des fluides ont une lumière identique ou des lumières différentes et les canaux (4, 4a, 4b, 5) permettent de conduire des fluides entre eux, et/ou dans lequel des canaux (4, 4a, 4b, 5) pris individuellement sont reliés, de façon permettant de conduire des fluides, à des segments absorbeurs de fluide (1) pris individuellement ou groupés.

6. Dispositif de traitement par pression négative selon l'une des revendications précédentes, dans lequel des canaux (4, 4a, 4b, 5) permettant de conduire des fluides sortent du drain effectif de quelques centimètres ou décimètres pour pouvoir servir de sonde d'alimentation.

7. Dispositif de traitement par pression négative selon l'une des revendications précédentes, dans lequel un fil-guide (3) peut être introduit dans un canal (4) et/ou dans lequel le tube de drainage (13) est pourvu d'une courbure (queue de cochon) à son extrémité distale.

8. Dispositif de traitement par pression négative selon l'une des revendications précédentes, présentant une longueur allant de 80 cm à 250 cm et un diamètre extérieur allant de 2 mm à 20 mm, des drains de petit calibre pouvant être introduits par le canal de travail d'un endoscope.

9. Dispositif de traitement par pression négative selon l'une des revendications précédentes, dans lequel des fils de métal ou autre matériau sont intégrés afin d'élever la résistance à la traction de l'élément de contact (1) et/ou de l'élément de communication fluidique (2) et dans lequel la résistance à la traction va jusqu'à 200 N et/ou dans lequel le drain est opaque aux rayons X et/ou dans lequel il est prévu, à l'extrémité distale du drain, un dispositif permettant de saisir le drain à l'aide d'instruments endoscopiques.

10. Dispositif de traitement par pression négative selon l'une des revendications précédentes, relié, par le biais d'éléments de raccord, à un système de production de vide, notamment une pompe à vide électronique, de façon permettant de conduire des fluides, un vide allant de 40 mmHg à 200 mmHg étant mis en œuvre.

11. Dispositif de traitement par pression négative selon l'une des revendications précédentes, comportant un corps creux tubulaire à usage médical dans le corps humain ou animal et dont la face extérieure consiste en l'élément de contact (1), ledit élément de contact (1) consistant en une membrane non perméable aux gaz et aux liquides dont la face tournée vers l'extérieur présente une surface (12) poreuse, le long de laquelle surface (12) peuvent s'écouler des liquides et/ou des gaz, et dont la face tournée vers l'intérieur présente une surface (11) non poreuse.

12. Dispositif de traitement par pression négative selon la revendication 11, dans lequel le corps creux consiste en un stent en treillis métallique et/ou plastique auto-expansible enveloppé au moins en partie par une feuille unilatéralement poreuse ou en un tuyau (15) tubulaire.

13. Dispositif de traitement par pression négative selon la revendication 11 ou 12, dans lequel la face à surface poreuse (22) est reliée, de façon permettant de conduire des fluides, à au moins un élément de communication fluidique (2) qui peut être relié, de façon permettant de conduire des fluides, à un système de production de pression négative, et dans lequel l'élément de communication fluidique (2) consiste en un tube (7) qui est de préférence détachable du corps creux.

14. Dispositif de traitement par pression négative selon l'une des revendications 11 à 13, dans lequel au moins un élément de communication fluidique (2) est relié, de façon permettant de conduire des fluides, à la face à surface poreuse (22) par une ouverture (21b) traversant la face à surface non poreuse (21).

15. Dispositif de traitement par pression négative selon l'une des revendications 11 à 14, dans lequel la surface poreuse (22) présente une structure de type treillis, filet, bulles, bosselages, doigts ou canal.

16. Dispositif de traitement par pression négative selon l'une des revendications 11 à 15, dans lequel le corps creux consiste en un tuyau (15) tubulaire dont la paroi est conçue, au moins en partie, unilatéralement poreuse, la face extérieure dudit tuyau (15) étant conçue sous la forme d'une surface poreuse (22) présentant les propriétés de porosité de la feuille poreuse, et sa face intérieure étant non poreuse.

17. Dispositif de traitement par pression négative selon l'une des revendications 11 à 16, dans lequel le corps creux présente un évasement en entonnoir à une ou aux deux extrémités.

18. Dispositif de traitement par pression négative selon l'une des revendications 11 à 17, dans lequel le tuyau (15) consiste en un tuyau d'intubation à une ou deux lumières.

19. Dispositif de traitement par pression négative selon l'une des revendications 11 à 18, dans lequel la face à surface poreuse (22) de la feuille est reliée, par au moins un élément de communication fluidique (2), à un système de production de vide conçu pour appliquer sur la surface (22) de la feuille poreuse une pression négative pouvant aller jusqu'à 200 mmHg.

20. Dispositif de traitement par pression négative selon l'une des revendications 11 à 19, dans lequel la porosité de ladite face de la feuille résulte d'un traitement mettant en œuvre un élément poreux absorbeur de fluide et/ou dans lequel l'élément absorbeur de fluide se compose d'une mousse en polyuréthane poreuse et dans lequel la taille des pores de la surface poreuse est comprise entre 200 µm et 1000 µm.
